# EUROPEAN PATENT APPLICATION

(11) **EP 2 055 717 A1**
(43) Date of publication of application: **06.05.2009**
(21) Application number: 07119786.7
(22) Date of filing: 31.10.2007
(51) Int. Cl.: C07K 16/00, C12N 9/08, G01N 33/574

(54) **Prognostic method**

(71) Applicant: Atlas Antibodies AB, 106 91 Stockholm (SE)
(72) Inventor: Uhlén, Mathias, 182 79 Stocksund (SE); Pontén, Fredrik, 752 37 Uppsala (SE)
(74) Representative: Pierrou, Mattias

(57) **Abstract**

The invention provides new methods, means and uses in connection with establishing a prognosis for cancer patients. The provided method for determining whether a prognosis for a mammalian subject having or suspected of having a cancer is worse than or equal to a reference prognosis comprises the steps of: providing a sample earlier obtained from the subject; evaluating the amount of GPX2 protein present in at least part of the sample and determining a sample value corresponding to the amount; comparing the obtained sample value with a reference value associated with the reference prognosis; and, if the sample value is equal to or lower than the reference value, concluding that the prognosis for the subject is equal to or worse than the reference prognosis.

## Description

### Field of the invention

The present invention relates to the field of cancer prognostics. In particular, it provides new means for establishing a prognosis for a patient having or suspected of having a cancer.

### Background of the invention

### Cancer

Cancer is one of the most common causes of disease and death in the western world. In general, incidence rates increase with age for most forms of cancer. As human populations continue to live longer, due to an increase of the general health status, cancer will affect an increasing number of individuals. The cause of most common cancer types is still at large unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. It is important to emphasize that a malignant tumor does not only consist of the transformed tumor cells themselves but also surrounding normal cells which act as a supportive stroma. This recruited cancer stroma consists of connective tissue, blood vessels and various other normal cells, e.g. inflammatory cells, which act in concert to supply the transformed tumor cells with signals necessary for continued tumor growth.

The most common forms of cancer arise in somatic cells and are predominantly of epithelial origin, e.g. prostate, breast, colon, urothelial and skin, followed by cancers originating from the hematopoetic lineage, e.g. leukemia and lymphoma, neuroectoderm, e.g. malignant gliomas, and soft tissue tumors, e.g. sarcomas.

### Cancer diagnostics and prognostics

Microscopic evaluation of a tissue section taken from a tumor remains the golden standard for determining a diagnosis of cancer. For microscopic diagnosis, biopsy material from suspected tumors is collected and examined under the microscope. To obtain a firm diagnosis, the tumor tissue is fixated in formalin, histo-processed and paraffin embedded. From the resulting paraffin block, tissue sections can be produced and stained using both histochemical, i.e. hematoxylin-eosin staining, and immunohistochemical methods. The surgical specimen is then evaluated with pathology techniques, including gross and microscopic analysis. This analysis forms the basis for assigning a specific diagnosis, i.e. classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e. how far the tumor has invaded and imposed growth into surrounding normal tissues, whereas the N stage and M stage describe how the tumor has developed into metastasis, with the N stage describing spread of tumor to lymph nodes and the M stage describing growth of tumor in other distant organs. Early stages include: T0-1 , N0, M0, representing localized tumors with negative lymph nodes. More advanced stages include: T1-4, N0-4, M0, localized tumors with more widespread growth and T1-4, N1-4, M0, tumors that have metastasized to lymph nodes and T1-4, N1-4, M1, tumors with a metastasis detected in a distant organ. Staging of tumors is based on several forms of examinations, including surgical, radiological and pathological analyses.

Accurate staging is crucial for a correct diagnosis and provides an instrument to predict prognosis. The diagnostic and prognostic information for a specific tumor subsequently determines an adequate therapeutic strategy for the given cancer patient. The most commonly used method, in addition to histochemical staining of tissue sections, to obtain more information regarding a tumor is immunohistochemistry. Immunohistochemistry allows for the detection of protein expression patterns in tissues and cells using specific antibodies. The use of immunohistochemistry in clinical diagnostics allows for the detection of immunoreactivity in different cell populations, in addition to the information regarding tissue architecture and cellular morphology that is assessed from the histochemically stained tumor tissue section. Immunohistochemistry can be important to support the accurate diagnosis, including staging and grading, of a primary tumor as well as in the diagnostics of metastases of unknown origin. The most commonly used antibodies in clinical practice today include antibodies against cell type "specific" proteins, e.g. PSA (prostate), MelanA (melanocytes), Thyroglobulin (thyroid gland) and antibodies recognizing intermediate filaments (epithelial, mesenchymal, glial) cluster of differentiation (CD) antigens (hematopoetic, sub-classification of lympoid cells) and markers of malignant potential, e.g. Ki67 (proliferation), p53 (commonly mutated tumor suppressor gene), HER-2 (growth factor receptor).

Aside from immunohistochemistry, the use of *in situ* hybridization for detecting gene amplification and gene sequencing for mutation analysis are evolving technologies within cancer diagnostics. In addition, global analysis of transcripts, proteins or metabolites all add important information. However, most of these analyses still represent basic research and have yet to be evaluated and standardized for the use in clinical medicine.

In order for doctors to give a cancer patient the right type of treatment as early as possible, the provision of new molecular markers that allows for more accurate stratification of cancer patients into different risk categories is crucial. We are still far a way from this type of individually tailored treatment. In summary, there is a great demand for new means to advance prognostics and staging of cancer.

### Malignant epithelial tumors

Malignant epithelial tumors are tumors showing an epithelial phenotype, i.e. tumor cells express characteristic intermediate filaments etc that signify epithelial differentiation. Epithelial cancers originate from organs and tissues where epithelial cells play a major functional role, e.g. surface epithelia and glands. The main forms are adenocarcinoma, squamous cell carcinoma and transitional cell carcinoma. Adenocarcinomas thus show glandular type of differentiation and can originate from all forms of glandular tissues, e.g. the prostate, breast, gastrointestinal tract (from stomach to the anal mucosa), pancreas, bile ducts, lung, endometrium etc. Squamous cell carcinomas, showing squamous differentiation patterns, can arise either from organs normally covered by squamous epithelium, e.g. skin, oral mucosa, esophagus etc. or from tissues where glandular epithelia have undergone squamous metaplasia, e.g. lung, cervix etc. Transitional cell carcinomas arise from a specialized form of epithelia that is found as surface epithelia in the urinary system and also in parts of the airway system, e.g. nasal and sinusoidal mucosa. The other forms of malignant tumors, e.g. lymphoma, glioma and sarcoma arise from organs and tissues where the corresponding normal cells express other characteristic features that signify mesenchymal differentiation, e.g. vimentin in hematopoetic cells (lymphoma) and soft tissues (sarcoma) or glial differntiation, e.g. GFAP (glioma).

A malignant epithelial tumor, or carcinoma, grow invasively into surrounding normal environment, damaging and destroying normal structures and functions of normal tissues and organs in the vicinity. As the tumor continues to develop and grow there is an increased risk of the tumor forming metastasis, allowing for widespread growth of tumor at various locations of the body. Spread of tumor cells is thought to occur either through the lymphatic system, which gives rise to lymph node metastasis or via the blood system, which gives rise to metastatic growth in distant organs (most commonly liver, lung, bone marrow and brain). As for all forms of neoplasia, epithelial tumors are classified based on histopathological appearance. The two most common forms of malignant epithelial tumors are adenocarcinoma and squamous cell carcinoma. The used terms reflect the fact that tumor cells show glandular- or squamous-like features respectively. Carcinomas are staged according to the extent of disease. The TNM system is most often used. For some common tumors classic staging systems, such as the Dukes' classification for colon cancer (which at large is equivalent to the TNM staging of colon cancer), are still used.

### The gastrointestinal tract including associated organ systems

The gastrointestinal tract is the system of organs that involves digesting food to extract nutrients and expelling the remaining waste. Consequently, the major functions of the gastrointestinal tract are digestion, absorption and excretion. The function of the gastrointestinal tract relies on accessory organs to the gastrointestinal tract, which include the liver, gallbladder, and pancreas. The liver secretes bile into the small intestine via the biliary system, employing the gallbladder as a reservoir. The pancreas secretes an isosmotic fluid containing bicarbonate and several digestive enzymes into the small intestine.

Today, malignant epithelial tumors frequently arise in the gastrointestinal tract including associated organs, and such cancers are a major cause of death in the western world. The environments of various tissues of the gastrointestinal tract including associated organs have common components, such as components resulting from the digestion of food and components of the gastric, bile or isosmotic fluid, which may affect the development, progression and behavior of cancers.

### Adenocarcinomas from colon and rectum (colorectal cancer)

Colorectal cancer, a malignant epithelial tumor that presents as an adenocarcinoma, is one of the most common forms of human cancer worldwide. Data from the GLOBOCAM 2002 database presented by Parkin *et al* show that around 1 million new cases of colorectal cancer are identified yearly (Parkin et al (2007) CA Cancer J Clin 55, 74-108). Further, the incidence of colorectal cancer in the world is approximately 9.4 % of all cancers, and colorectal cancer constitutes the second most common cause of death in the western world. The five-year survival rate of colorectal cancer is approximately 60 % in the western world but as low as 30 % in Eastern Europe and India.

Early detection and surgery with excision of the tumor is currently of critical importance for a successful treatment. For localized tumors, i.e. tumors that have not evolved into a metastasizing disease, surgical intervention with radical resection of the tumor and surrounding bowel and tissues is performed. Colorectal tumors are categorized into several stages according to Dukes' stages A-C or more recently according to the TNM classification. The least malignant tumors (Dukes' stages A and B) are generally associated with a relatively favorable outcome, while highly malignant tumors with metastasis (Dukes' stage C and D) have poor survival rates. Unfortunately, colorectal cancer has often grown to a considerable size before detection and thus metastases are not uncommon. The tumor typically metastasizes to regional lymph nodes, but distant metastasis to the liver and lung are also common.

Symptoms depend on where in the distal gastrointestinal tract the tumor is located, and include bowel distress, diarrhea, constipation, pain and anemia (secondary to bleeding from the tumor into the bowel). Current diagnostics are based on patient history, clinical and endoscopic examination (rectoscopy and colonoscopy), optionally followed by radiological mapping to determine extensiveness of tumor growth. In conjunction with endoscopic examination, tissue biopsies are performed from dubious lesions.

In differential diagnostics, cytokeratin 20 (CK20), an intermediate filament marker abundant in the glandular cells of the GI-tract, is commonly used to diagnose primary tumors in the GI-tract including colorectal cancer. The CK20 marker is not ideal as several other adenocarcinomas also can be positive for CK20 antibodies, whereas not all colorectal cancers are positive. Prognostic information is mainly obtained from tumor staging classification as there are no accepted grading systems or protein markers that provide additional prognostic data. Today there are no available markers that can distinguish tumors of low malignancy grade and low risk for developing into a metastasizing disease from highly malignant tumors with a reduced chance of survival. There is thus a great need for molecular markers that can be used to predict patient outcome and to guide for patient management including therapeutic intervention.

### Transitional cell carcinoma from the urinary system (urothelial cancer)

Urothelial cancer is a malignant epithelial tumor in the urinary bladder. Today, urotheilial cancer is the ninth most common form of human cancer worldwide. Data from the GLOBOCAM 2002 database presented by Parkin *et al,* show that around 0.36 million new cases of urothelial cancers are identified yearly (Parkin et al (2007) CA Cancer J Clin 55, 74-108). Further, the incidence of urothelial cancer in the world is approximately 0.33 % of all cancers, with nearly 0.15 million death per year worldwide.

Early detection and surgery with excision of the tumor is of critical importance for a successful treatment. Superficial tumors can quite easily be surgically removed or "shaved off". If the tumor has reached into the deeper lying musculature of the urinary bladder and perhaps also grown beyond the urinary bladder into prostate, vagina, or uterus, a more radical operation is needed. Removal of the bladder may then be necessary in combination with chemotherapy. Bladder cancer typically metastasizes to regional lymph nodes, but distant metastasis in the lung, skin, liver and bones are not unusual. Blood in the urine is a common first symptom, but not always observed. Other symptoms may be pain across the pubic bone, frequent urination, and stinging or similar symptoms as in an ordinary bladder infection.

Current diagnostics are based on patient history, clinical and cystoscopic examination i.e. a flexible tube is introduced into the bladder through the urethra. The tube is bearing a camera and a tool to remove tissue from dubious lesions. Urothelial tumors are classified according to the TNM staging system and graded according to WHO standards. The least malignant tumors, Ta and T1, are associated with a relatively favorable outcome, a five-year survival rate of 90 and 75 %, respectively. More invasive tumors have a less favorably prognosis with a five-year survival rate of approximately 60 % for stage T2 and 35% for stage T3, corresponding to when the tumor reached beyond the urinary bladder wall musculature. Tumors with metastasis (N1-4 and/or M1) are associated with an even worse prognosis.

For differential diagnostics, there are no markers available today that are recommended for routinely clinical use. However, FDA has cleared a handful of markers e.g. BTA-Stat and NMP22 that can be used for screening and diagnosis. The problem using these markers is that they have a low sensitivity and specificity, in particular urine markers. Furthermore, markers associated to prognosis regarding urothelial cancer are lacking. Consequently there is a great need for new markers for bladder cancer prognostics.

### GPX2

The gene encoding glutathione peroxidase 2 (GPX2) was initially identified as an epithelium-specific gastrointestinal glutathione peroxidase (Chu et al (1993) J Biol Chem 5, 2571-2576). Expression was detected in colon and liver but also tumorous breast tissue. Studies of the gene and its protein product revealed a cytoplasmic location and involvement in inflammation and cancer. Up-regulation has been reported in response to oxidative stress i.e. during inflammatory bowel diseases (Chu et al (2004) Free Radic Res 36, 1481-95) and in patients with Barretts's oesopagus (Mörk et al (2003) Int J Cancer 20, 300-304). In addition, an increased level of both GPX2 transcripts and protein has been detected in early stages of colon cancer (Mörk et al (2000) Nutr Cancer 37, 108-116, Florian S et al (2001) Free Radic Res 35, 655-663). Moreover, levels of both GPX2 transcripts and protein have been studied in colorectal adenocarcinomas (Florian S et al (2001) Free Radic Res 35, 655-663, Lin et al (2002) Oncogene 21, 4120-4128, AI-Taie et al (2004) Nutr Cancer 48, 6-14). The authors conclude that more research is needed to understand the results from the studies.

A clinical value of estimating the level of GPX2 in patients has been reported at several occasions such as in patent WO0200939. GPX2 is here presented as a "colon specific gene" that can be used for diagnosing the presence of colon cancer. A change in the level compared to the level in a control sample, i.e. tissue sample from a normal human colon, is associated with the presence of colon cancer. Also, a method for staging colon cancer is presented where an increase of the level of GPX2 is associated with progression of the disease and a decrease of the GPX2 level is associated with a cancer that is regressing or in remission. They further suggest that treating colon cancer could be done with a compound that will down-regulate expression or activity of GPX2. In patent WO02004/019037, the authors demonstrate the usefulness of investigating body fluids for cancer cells. Detecting high levels of enzymes with an oxidative function, such as members of the GPX protein family, is according to the authors an indication of the presence of disseminated cancer cells in the body fluid and is accordingly a way for diagnosis of a tumor. An elevated enzyme level would indicate the presence of a tumor and could also be used to estimate the risk to develop a metastasis or a recurrence. In patent WO2004/033641, genes related to bladder cancer are listed. The level of GPX2 transcripts, such as those of many other transcripts, was examined when analyzing global gene expression of superficial versus invasive bladder cancer. However, the authors found other transcripts more interesting, and GPX2 transcripts were not chosen for further studies.

### Disclosure of the invention

Importantly, none of the studies referred to above suggests low GPX2 protein levels as an indicator of a poor prognosis for a cancer patient. Neither do the studies suggest high GPX2 protein levels as an indicator of a good prognosis for a cancer patient.

Summarizing the state of the art, there is a great need for new tools for cancer prognostics.

It is an object of the present invention to meet this demand through the provision of improvements relating to prognostics for a subject having or suspected of having a cancer.

For this and other objects apparent to the skilled person from the present disclosure, the present invention provides, in its different aspects, new means for determining a prognosis for a subject having or suspected of having a cancer, and for the treatment thereof. The present invention is defined by the appending claims.

Thus, according to a first aspect, the present invention provides a method for determining whether a prognosis for a mammalian subject having or suspected of having a cancer is worse than a reference prognosis, comprising the steps of:
a) providing a sample earlier obtained from the subject;
b) evaluating the amount of GPX2 protein present in at least part of said sample and determining a sample value corresponding to said amount;
c) comparing the sample value obtained in step b) with a reference value associated with said reference prognosis; and, if said sample value is equal to or lower than said reference value,
d) concluding that the prognosis for said subject is equal to or worse than said reference prognosis.

Regarding step b), an increase in the amount of GPX2 protein typically results in an increase in the sample value, and not the other way around.

In an embodiment, the above method may comprise the additional step:
e) if said sample value is higher than said reference value, concluding that the prognosis for said subject is better than said reference prognosis.

This first aspect of the present invention is based on the previously unrecognized fact that the expression of GPX2 protein in samples earlier obtained from a subject having or suspected of having a cancer may serve as an indicator of disease status in subjects. More particularly, the present invention identifies for the first time, in patients suffering from a cancer, a correlation between a value of GPX2 protein expression on the one hand and a prognosis for survival on the other. Typically, low GPX2 values have been shown to correlate with a poor prognosis in these patients, probably due to a more aggressive or high-risk form of the cancer. The present invention based on GPX2 protein expression as an indicator of cancer status has a number of benefits. In general, early identification of aggressive forms of a cancer is of vital importance as it enables curing treatment. As an example, referring to colorectal cancer, for which several large studies have shown that subjects with early cancers, i.e. representing stage 1 and stage 2 tumors (essentially Dukes' A and B), have a substantially better prognosis as compared to subjects with late stage tumors. This difference is not dependent on the mode of treatment since radical resection is performed for all types of colorectal cancer. Rather, the large difference in survival is clearly related to early detection, correct diagnosis and adequate surgical treatment. The GPX2 protein, as a marker for which a certain level of expression is correlated with a certain pattern of disease progression, has a great potential for example in a panel for differential diagnostics of a primary tumor.

In the present disclosure, different GPX2 values (sample values) corresponding to various prognoses are presented. Typically, a low sample value is associated with a poorer prognosis than a high sample value. In the above method, the sample value is compared to a reference value, and if the sample value is equal to, or lower than, the reference value, it is concluded that the prognosis for the subject is equal to, or worse than, a reference prognosis associated with the reference value.

Consequently, the above method may be adapted to a given reference value. In such case, starting from a given sample value which under certain circumstances is considered to be relevant, a reference value which is equal to, or higher than, that sample value, may be selected. Subsequently, a reference prognosis being associated with that reference value may be established. Guided by the present disclosure, the person skilled in the art understands how to establish a reference prognosis which corresponds to a given reference value. For example, the relation between sample values and survival data in a group of cancer patients may be examined as in Examples, section 4, below, and the procedure described therein may be adapted to a given reference value. Then, a prognosis corresponding to the given reference value may be selected as the reference prognosis.

Also, the above method may be adapted to a given reference prognosis. In such case, starting from a given reference prognosis which under certain circumstances is considered to be relevant, for example for selecting an appropriate therapy, a corresponding reference value may be established. Guided by the present disclosure, the person skilled in the art understands how to establish a reference value which corresponds to a given reference prognosis. For example, the relation between sample values and survival data in a group of cancer patients may be examined as in Examples, section 4, below, but the procedure described therein is adapted to establish reference values corresponding to a given reference prognosis. For example, different reference values may be tested until one which correlates with the given reference prognosis is found.

In embodiments of the above method aspect, the reference prognosis may be a likelihood of five-year survival of 66 % or lower, for example 65 % or lower, 60 % or lower, 50 % or lower, 40 % or lower, 30 % or lower, 20 % or lower, 10 % or lower, or 0 %.

As an example, the reference prognosis may be an average five-year survival for patients having the cancer, such as the average five-year survival for patients having the cancer at the time of performing the method. For example, the average five-year survival of a patient diagnosed with colorectal cancer was about 60 % world wide in 2002 (Parkin et al (2007) CA Cancer J Clin 55, 74-108), the average five-year survival of a patient diagnosed with stomach cancer in U.S. between 1985 and 1996 was less than 30% (Hundahl el al (2000) Cancer 88, 921-32), the average five-year survival of a patient diagnosed with pancreatic cancer was only a few percent, typically less than 10 %, and the average five-year survival of a patient diagnosed with urothelial cancer ranged between 40-80% in 2002 (Parkin et al (2007) CA Cancer J Clin 55, 74-108). Increased knowledge, development of new therapeutic agents and improved diagnostics will eventually increase the chance for prolonged survival for these patients. Consequently, due to the progression of the medical art, the survival numbers above may be higher in the future, such as in the second or third decade of the 21^{st} century.

Further, the reference prognosis may be based on a previous prognosis obtained by an examination of the same patient or population of patients. Alternatively, or as a complement, the reference prognosis may be based on a background risk in the general population, a statistical prognosis/risk or an assumption based on an examination of the patient. Such examination may comprise the patient's age, general condition, sex, race and/or medical status and history, such as cancer history or cancer status. For example, a reference prognosis may be established by a physician taking into regard the patient's type of cancer and cancer history, the stage of the tumor, the morphology of the tumor, the location of the tumor, the presence and spread of metastases and/or further cancer characteristics.

In an alternative embodiment of the first aspect, the present invention provides a method for determining whether a prognosis for a mammalian subject having or suspected of having a cancer is poor, comprising the steps of:
a1) providing a sample earlier obtained from the subject;
b1) evaluating the amount of GPX2 protein present in at least part of said sample to yield a sample value;
c1) comparing the sample value obtained in step b1) with a reference value; and, if said sample value is equal to or lower than said reference value,
d1) concluding that the prognosis for said subject is poor.

Regarding step b1), an increase in the amount of GPX2 protein typically results in an increase in the sample value, and not the other way around.

In an embodiment of the alternative embodiment of the first aspect, the method may comprise the additional step:
e1) if said sample value is higher than said reference value, concluding that the prognosis for said subject is not poor.

In an embodiment, the conclusion in step d1) of a poor prognosis may involve establishing that said subject has a shorter expected survival time than would have been the case if the subject had not exhibited a low GPX2 protein expression value. Alternatively, or as a complement, the conclusion may be that said subject has a lower likelihood of survival, such as five-year survival or ten-year survival, than would have been the case if the subject had not exhibited a low GPX2 protein expression value. For example, the conclusion may be that said subject has a likelihood of five-year survival of 66 % or lower, for example 65 % or lower, 60 % or lower, 50 % or lower, 40 % or lower or 30 % or lower. As an example, regarding subjects having or suspected of having colorectal cancer, the conclusion may be that said subject has a likelihood of five-year survival of 66 % or lower, for example 65 % or lower, 60 % or lower, 50 % or lower, 40 % or lower or 30 % or lower. As another example, regarding subjects having Dukes' stage C colorectal cancer, the conclusion may be that said subject has a likelihood of five-year survival of 46 % or lower, for example 40 % or lower, 30 % or lower, 20 % or lower, 15 % or lower, 10 % or lower, 5 % or lower or 2 % or lower.

What to consider as a poor prognosis may change over time because the art of medicine is progressing. At any given occasion, what to be considered as a poor prognosis for a patient having a cancer may be determined with regard to the normal prognosis for that cancer at that time. As an example, a poor prognosis for a certain type of cancer may be a prognosis that is poorer than the average prognosis for that type of cancer at the time by which the method is performed.

Accordingly, a poor prognosis may be a likelihood of a five-year survival which is lower than the average five-year survival at the time of performing the method. For example, the average five-year survival of a patient diagnosed with colorectal cancer was about 60 % world wide in 2002 (Parkin et al (2007) CA Cancer J Clin 55, 74-108), the average five-year survival of a patient diagnosed with stomach cancer in U.S. between 1985 and 1996 was less than 30% (Hundahl el al (2000) Cancer 88, 921-32), the average five-year survival of a patient diagnosed with pancreatic cancer was only a few percent and the average five-year survival of a patient diagnosed with urothelial cancer ranged between 40-80% in 2002 (Parkin et al (2007) CA Cancer J Clin 55, 74-108). Increased knowledge, development of new therapeutic agents and improved diagnostics will eventually increase the chance for prolonged survival for these patients. Due to the progression of the medical art, these numbers may be higher in the future, such as in the second or third decade of the 21^{st} century.

Consequently, in one embodiment, the poor prognosis in step d1) corresponds to a likelihood of five year survival which is lower than the average five-year survival for the cancer at the time of performing the method.

As an example, the cancer may be a previously established type of cancer, and the poor prognosis in step d1) corresponds to a likelihood of five year survival which is lower than the average five-year survival for the previously established type of cancer at the time of performing the method.

As another example, the cancer is a previously established type of cancer in a previously established stage, and the poor prognosis in step d) corresponds to a likelihood of five year survival which is lower than the average five-year survival for the previously established type of cancer in the previously established stage at the time of performing the method.

The identified correlation between low GPX2 protein expression and high-risk forms of a cancer may also form the basis for a decision to apply a specific regime for treatment of the subject. Thus, in a second aspect, the present invention provides a method of treatment of a subject being in need thereof, wherein the subject is having a cancer, comprising:
a) providing a sample from the subject;
b) evaluating the amount of GPX2 protein present in at least part of said sample, and determining a sample value corresponding to said amount;
c) comparing the sample value obtained in step b) with a reference value associated with a reference prognosis; and, if said sample value is equal to or lower than said reference value,
d) treating said subject with a treatment regimen adapted to a prognosis which is worse than or equal to the reference prognosis.

Regarding step b), an increase in the amount of GPX2 protein typically results in an increase in the sample value, and not the other way around.

In an embodiment of the second aspect, the method may comprise the additional step:
e) and if said sample value is higher than said reference value, not treating said subject with a cancer treatment regimen or treating said subject with a treatment regimen adapted to a prognosis which is better than the reference prognosis.

In one embodiment, the treatment regimen of step d) is selected from chemotherapy, neo-adjuvant therapy and combination thereof.

Thus, the treatment regimen may be neo-adjuvant therapy. Such neo-adjuvant therapy may consist of radiation therapy only or of radiation therapy in combination with chemotherapy.

Further, the treatment may be chemotherapy.

In embodiments of the methods of the above aspects, the sample may be a body fluid sample, such as blood, plasma, serum, cerebral fluid, urine, semen and exudate. Alternatively, the sample may be a cytology sample or a stool sample.

In further embodiments of the methods of the above aspects, the sample may be a tissue sample.

In one embodiment, the tissue sample is derived from the gastrointestinal tract including accessory organs of the subject. In the context of the present disclosure, the "gastrointestinal tract including accessory organs" refers to the gastrointestinal tract and the liver, gallbladder, and pancreas. Consequently, in this context, the accessory organs refer to the liver, gallbladder and pancreas. Further, in the context of the present disclosure, the "gastrointestinal tract" refers to the system within multicellular organisms digests food to extract energy and nutrients and expels the remaining waste. For example, the stomach, intestines, such as the small intestine, duodenum, appendix, colon and rectum, and anus belongs to the gastrointestinal tract.

For example, the tissue sample may be derived from the gastrointestinal tract.

In embodiments of the methods of the above aspects, the sample may be a tumor tissue sample.

In embodiments of the methods of the above aspects, the tissue sample may be a stomach sample, a pancreas sample, a liver sample, a colorectal sample, or a urothelial sample. Also, the tissue sample may be derived from a group of organs consisting of stomach, pancreas, liver, colon, rectum, and urinary bladder. Further, the tissue sample may be derived from a group of organs consisting of stomach, pancreas, liver, colon and rectum. Alternatively, the tissue sample may be derived from a group of organs consisting of colon, rectum and urinary bladder. See below for a discussion about cancer and GPX2 protein expression in different types of organs and tissues.

In embodiments of the methods of the above aspects, the sample may comprise epithelial cells, such as glandular cells or transitional cells, from the subject. As can be seen in Examples, section 3, table 1 below, in normal tissue, the GPX2 protein is mainly expressed in glandular cells or surface epithelial cells. Further, GPX2 protein is for example expressed in glandular cells from cancer tissues, such as tissue from colorectal cancers, pancreatic cancers and stomach cancers, and in epithelial cells, i.e. transitional cells, from urothelial cancers. Consequently, samples comprising epithelial cells, such as glandular cells or epithelial cells, may suitably be evaluated for GPX2 protein expression.

In malignant epithelial cells, such as glandular cells and transitional cells, including adenocarcinoma cells, the GPX2 protein is mainly expressed in the cytoplasm. Consequently, the evaluation of GPX2 expression in a sample may be limited to an analysis of cytoplasmic expression in tumor cells present in the sample, e.g. a tumor tissue biopsy material. As an example, the evaluation of step b) or b1) in the above methods, may be limited to evaluating the amount GPX2 protein in the cytoplasms of epithelial cells, such as glandular cells or transitional cells, of said sample.

It is contemplated that the inventive method will be used in connection with many different types of cancers. This is supported by e.g. that GPX2 protein is expressed in a wide variety of normal tissue as can be seen in table 1.

Further, groups of subjects having malignant epithelial tumors, such as stomach cancer, pancreatic cancer, colorectal cancer, urothelial cancer or liver cancer, exhibit similar GPX2 protein expression patterns: samples from some of the subjects in a group show a strong positivity for GPX2 protein, whereas samples from other subjects in the group show moderate, weak or even no positivity (Examples, section 3, table 2). For example, all cancer types derived from the gastrointestinal tract including associated organs tested, i.e. stomach cancer, pancreatic cancer, colorectal cancer and liver cancer, showed such pattern (table 2). Further, the inventors have found that for two types of cancers affecting different types of tissue, a low GPX2 expression is correlated to a poor prognosis for the cancer type. Consequently, it is reasonable to believe that a corresponding relationship exists for other cancers as well, especially for malignant epithelial tumors, such as stomach cancer, pancreatic cancer, and liver cancer which show similar GPX2 expression patterns as colorectal cancer and urothelial cancer.

Consequently, the method aspects described above concerns a mammalian subject having or suspected of having a cancer. In embodiments of the methods of the above aspects, the cancer may be a malignant epithelial tumor. Examples of malignant epithelial tumors are colorectal cancer, stomach cancer, pancreatic cancer, urothelial cancer and liver cancer.

For example, the malignant epithelial tumor may be a malignant tumor in the gastrointestinal tract including accessory organs. Examples of malignant tumors in the gastrointestinal tract including accessory organs are colorectal cancer, stomach cancer, pancreatic cancer and liver cancer. The malignant tumor in the gastrointestinal tract including accessory organs may be a malignant tumor in the gastrointestinal tract, such as an adenocarcinoma in the gastrointestinal tract. Examples of such cancers are colorectal cancer and stomach cancer.

As an example, the cancer in question may be colorectal cancer, and the colorectal cancer may be in different forms and/or stages. In some embodiments, the colorectal cancer is a node-negative colorectal cancer, i.e. colorectal cancer that has not progressed to the lymph node metastasizing stage. In other similar embodiments, the colorectal cancer in question is characterized as being in either Dukes' stage A or B. In yet other embodiments, the colorectal cancer in question is colorectal adenoma or colorectal carcinoma. In these embodiments, determining that the subject exhibits low GPX2 expression may be of great value for the prognosis of future progression of the disease and thus form the basis for an informed decision with regard to future disease management.

Within a group of subjects afflicted with a comparatively early stage of disease, such as node negative colorectal cancer or Dukes' stage A or B colorectal cancer, subjects with low GPX2 expression likely are at a comparatively high risk of developing a more aggressive disease. Such expression may therefore indicate that these subjects should be monitored more closely and/or treated differently than subjects that do not exhibit low GPX2 expression. The methods according to the invention therefore offers the possibility of a greater chance for survival over a certain period of time and/or longer survival time for such subjects, owing to the additional prognostic information given by the GPX2 marker.

In other embodiments, the colorectal cancer is metastasizing colorectal cancer. In other similar embodiments, the colorectal cancer is characterized as being in Dukes' stage C.

In other embodiments, the colorectal cancer is selected from the group consisting of Dukes' stage A cancer and Dukes' stage B cancer.

As discussed in Examples, section 5 below, the inventors have shown that low GPX2 protein expression is correlated to a poor prognosis for subjects suffering from urothelial cancer. Consequently, in an embodiment, the malignant epithelial tumor may be a urothelial cancer.

As discussed above, groups of patients having liver cancer, stomach cancer or pancreatic cancer showed a similar GPX2 expression pattern as colorectal cancer and urothelial cancer. Consequently, in one embodiment, the cancer in question is selected from a group consisting of colorectal cancer, urothelial cancer, liver cancer, stomach cancer and pancreatic cancer. Further, the cancer in question may be selected from the group consisting of colorectal cancer, liver cancer, stomach cancer and pancreatic cancer. Alternatively, the cancer in question may be selected from the group consisting of colorectal cancer and urothelial cancer.

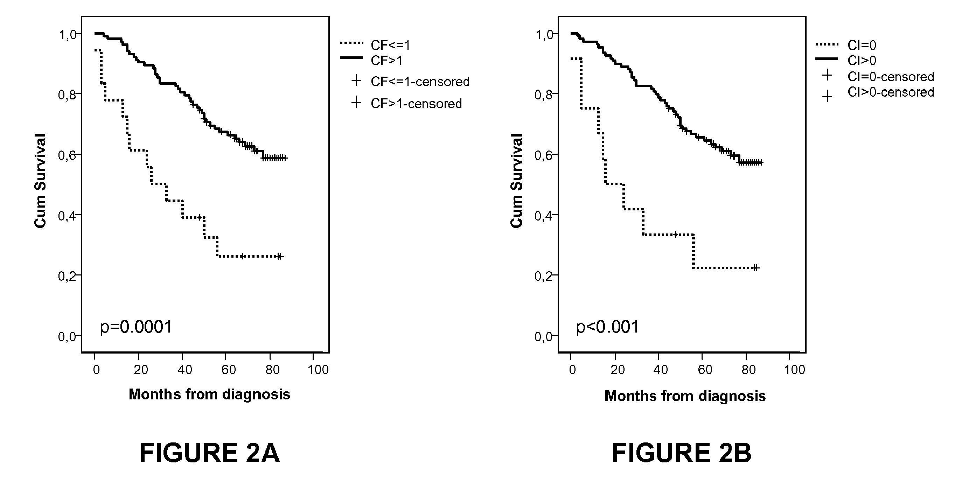

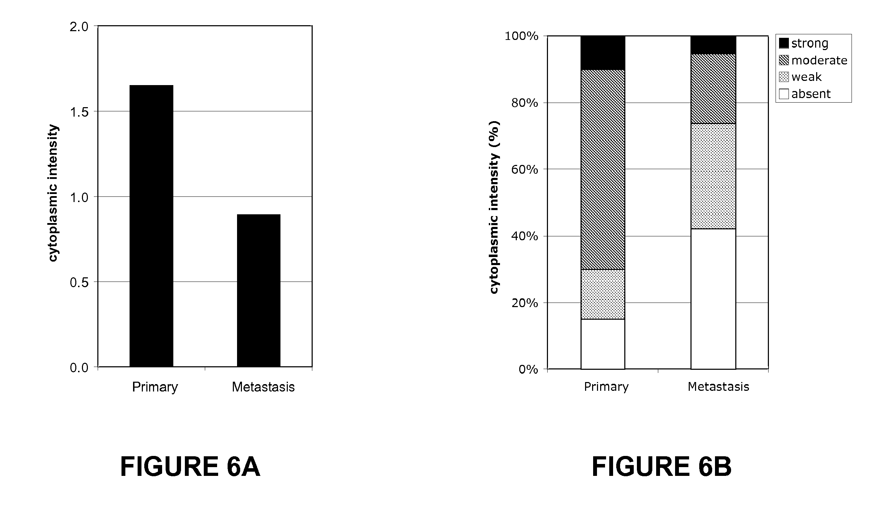

In another embodiment, the cancer in question affects a tissue, which normally expresses GPX2 protein, but loses or has significantly reduced GPX2 expression when affected by a cancer associated with a poor prognosis. For example, such tissue may be urothelial tissue, which normally expresses GPX2 protein (table 1), but when affected by cancer associated with a poor prognosis, exhibit a significantly reduced expression (figure 6). As another example, the tissue may be colon tissue (table 1; figures 2A and 2B).

A determination that the sample value of GPX2 protein expression is lower than the reference value is sometimes referred to herein as a determination of "low GPX2 protein expression".

In embodiments of the methods of the above aspects, the reference value for use as comparison with the sample value for a subject may be established in various ways. As one non-limiting example, the reference value may correspond to the amount of GPX2 protein expression in healthy tissue of the subject undergoing the prognosis or therapy. As another example, the reference value may be provided by the amount of GPX2 protein expression measured in a standard sample of normal tissue from another, comparable subject. As another example, the reference value may be provided by the amount of GPX2 protein expression measured in a reference sample comprising tumor cells, such as a reference sample of tumor tissue. As another example, the reference value may be provided by the amount of GPX2 protein measured in a reference sample comprising cells expressing a predetermined amount of GPX2 protein.

As another example the reference value may be provided by the amount of GPX2 protein expression measured in a reference sample comprising cancer cell lines expressing a predetermined, or controlled, amount of GPX2 protein. The person skilled in the art understands how to provide such cell lines, for example guided by the disclosure of Rhodes et al. (2006) The biomedical scientist, p 515-520.

Accordingly, in embodiments of the methods of the above aspects, the reference value may be a predetermined value corresponding to the amount of GPX2 protein in a reference sample.

In embodiments of the methods of the above aspects, the sample value of step b) may be either 1, corresponding to detectable GPX2 protein in the sample, or 0, corresponding to essentially no detectable GPX2 protein in the sample. Consequently, in such embodiment, the evaluation of the sample is digital: GPX2 protein is considered to be either present or not. In the context of the present disclosure, "essentially no detectable GPX2 protein" refers to an amount of GPX2 protein that is so small that it is not, during normal operational circumstances, detectable by a person or an apparatus performing the invention according to any one of its different aspects. The "normal operational circumstances" refer to the laboratory methods and techniques a person skilled in the art would find appropriate for performing the invention. Patients suffering from a cancer, such as colorectal cancer, who have lost essentially all their GPX2 protein expression, generally have a very poor prognosis.

In embodiments of the methods of the above aspects, the reference value of step c) may be provided by a reference sample, such as a tissue sample, having essentially no GPX2 protein expression.

In embodiments of the methods of the above aspects, the reference value of step c) may be 0, corresponding to essentially no detectable GPX2 protein.

In the context of the present disclosure, the terms "sample value" and "reference value" are to be interpreted broadly. The quantification of GPX2 protein to obtain these values may be done via automatic means, via a scoring system based on visual or microscopic inspection of samples, or via combinations thereof. However, it is also possible for a skilled person, such as a person skilled in the art of histopathology, to determine the sample and reference values merely by inspection of e.g. tissue slides that have been stained for GPX2 expression. The determination of the sample value being lower than the reference value may thus correspond to the determination, upon visual or microscopic inspection, that a sample tissue slide is less densely stained and/or exhibit fewer stained cells than is the case for a reference tissue slide. In this case, the sample and reference values are thought of as mental values that the skilled person determines upon inspection and comparison.

For example, the skilled person may categorize a sample as being positive or negative for GPX2 expression based on a previous inspection or an imagination of a reference sample lacking GPX2 protein expression. Alternatively, the skilled person may categorize a sample as being high or low in GPX2 protein expression, wherein the sample is categorized as low in GPX2 protein expression if it contains less GPX2 protein than a previously inspected reference sample. Such evaluation may be assisted by staining the sample, and, if necessary, the reference sample, with a staining solution comprising e.g. antibodies specific for GPX2 protein.

Thus, the invention is not limited to the use of automatic analysis.

One alternative for the quantification of GPX2 expression in a sample is the determination of the fraction of cells in the sample that exhibit GPX2 expression over a certain level. This determination may for example be performed as described below in the Examples, section 3 and section 4, definition of "cytoplasmic fraction". In embodiments of the methods of the above aspects, the criterion for the conclusion in step d) is a sample value for the nuclear fraction of GPX2 positive cells, i.e. a "cytoplasmic fraction", which is lower than the reference value of 50 %, such as lower than 40 %, such as lower than 30 %, such as lower than 25 %, such as lower than 20 %, such as lower than 15 %, such as lower than 10 %, such as lower than 5 %, such as lower than 1 %.

Further, the conclusion that the prognosis is equal to, or worse than, a reference prognosis may correspond to a detection of essentially no GPX2 positive cells in a sample, i.e. a "cytoplasmic fraction" of essentially zero. In alternative or complementing embodiments of the methods of the above aspects, the reference value of step c) is a cytoplasmic fraction of 50 % GPX2 positive cells or lower, such as 40 % GPX2 positive cells or lower, such as 30 % GPX2 positive cells or lower, such as 25 % GPX2 positive cells or lower, such as 20 % GPX2 positive cells or lower, such as 10 % GPX2 positive cells or lower, such as 5 % GPX2 positive cells or lower, such as 1 % GPX2 positive cells or lower.

Another alternative for the quantification of GPX2 expression in a sample is the determination of the over-all staining intensity of the sample. This determination may for example be performed as described below in the Examples, section 3 and section 4, definition of "cytoplasmic intensity". In embodiments of the methods of the above aspects, the criterion for the conclusion in step d) may be a sample value for staining intensity of a sample, i.e. a cytoplasmic intensity, which is equal to, or lower than, a moderate cytoplasmic intensity, such as equal to, or lower than, a faint cytoplasmic intensity, such as equal to no cytoplasmic intensity. In alternative or complementing embodiments of the methods of the above aspects, the reference value of step c) may be a moderate cytoplasmic intensity of GPX2 protein expression, or lower, such as a faint cytoplasmic intensity of GPX2 protein expression, or lower, such as no cytoplasmic intensity of GPX2 protein expression.

Guided by the present disclosure, and especially Examples, sections 3 and 4 below, a person skilled in the art, e.g. a pathologist, understands how to perform the evaluation yielding a "cytoplasmic fraction" or a "cytoplasmic intensity". In the evaluation, the skilled artisan may use a reference sample for establishing the appearance of a sample with a high amount of GPX2 protein. Such reference sample may be a sample comprising tissue expressing a high amount of GPX2, such as normal epithelial tissue from urine bladder. Referring to "cytoplasmic fraction", the reference sample may provide examples of cells with moderate or strong immunoreactivity (positive staining) and/or an example of a sample with more than 25 % positive cells. Referring to "cytoplasmic intensity", the reference sample may provide an example of a strong immunoreactivity.

Also referring to "cytoplasmic intensity", the reference sample may provide an example of a strong immunoreactivity. With the knowledge of the appearance of a sample with strong immunoreactivity (= distinct and strong immunoreactivity), the skilled artisan may then divide samples into the categories presented in Examples, section 3, below, i.e. absent (=no immunoreactivity), weak (= faint immunoreactivity), moderate (= medium immunoreactivity) and strong (= distinct and strong immunoreactivity). Further, referring to "cytoplasmic fraction", the reference sample may provide an example of a sample with e.g. more than 75 % positive cells. With the knowledge of the appearance of a sample with more than 75 % positive cells, the skilled artisan may then evaluate the "cytoplasmic fraction" of other samples having e.g. a lower percentage of positive cells.

The skilled person will recognize that the usefulness of the present invention is not limited to the quantification of any particular variant of the GPX2 protein present in the subject in question, as long as the protein is encoded by the relevant gene and presents the relevant pattern of expression. As a non-limiting example, the GPX2 protein has an amino acid sequence which comprises a sequence selected from:
i) SEQ ID N0:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the GPX2 protein has an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

In embodiments of the methods of the aspects above, the GPX2 protein may be detected and/or quantified through the application to a sample of a detectable and/or quantifiable affinity ligand, which is capable of specific or selective interaction with the GPX2 protein. The application of the affinity ligand is performed under conditions that enable binding of the affinity ligand to any GPX2 protein in the sample. It is regarded as within the capabilities of those of ordinary skill in the art to select or manufacture the proper affinity ligand and to select the proper format and conditions for detection and/or quantification, once the connection between GPX2 and cancer is known through the teaching of the present disclosure. Nevertheless, examples of affinity ligands that may prove useful, as well as examples of formats and conditions for detection and/or quantification, are given below for the sake of illustration.

Thus, in some embodiments, an affinity ligand is used, which is selected from the group consisting of antibodies, fragments thereof and derivatives thereof, i.e. affinity ligands based on an immunoglobulin scaffold. Antibodies comprise monoclonal and polyclonal antibodies of any origin, including murine, human and other antibodies, as well as chimeric antibodies comprising sequences from different species, such as partly humanized mouse antibodies. Polyclonal antibodies are produced by immunization of animals with the antigen of choice, whereas monoclonal antibodies of defined specificity can be produced using the hybridoma technology developed by Köhler and Milstein (Köhler G and Milstein C (1976) Eur. J. Immunol. 6:511-519). Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al (1991) Biochemistry 30:6803-6805); camelid heavy-chain dimers (Hamers-Casterman C et al (1993) Nature 363:446-448) and single variable domains (Cai X and Garen A (1996) Proc. Natl. Acad. Sci. U.S.A. 93:6280-6285; Masat L et al (1994) Proc. Natl. Acad. Sci. U.S.A. 91:893-896), and single domain scaffolds like e.g. the New Antigen Receptor (NAR) from the nurse shark (Dooley H et al (2003) Mol. Immunol. 40:25-33) and minibodies based on a variable heavy domain (Skerra A and Plückthun A (1988) Science 240:1038-1041).

Polyclonal and monoclonal antibodies, as well as their fragments and derivatives, represent the traditional choice of affinity ligands in applications requiring selective biomolecular recognition, such as in the detection and/or quantification of GPX2 protein according to the method aspects above. However, those of skill in the art know that, due to the increasing demand of high throughput generation of specific binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific and/or selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against GPX2 for use according to the present disclosure, are staphylococcal protein A and domains thereof and derivatives of these domains, such as protein Z (Nord K et al (1997) Nat. Biotechnol. 15:772-777); lipocalins (Beste G et al (1999) Proc. Natl. Acad. Sci. U.S.A. 96:1898-1903); ankyrin repeat domains (Binz HK et al (2003) J. Mol. Biol. 332:489-503); cellulose binding domains (CBD) (Smith GP et al (1998) J. Mol. Biol. 277:317-332; Lehtiö J et al (2000) Proteins 41:316-322); Y crystallines (Fiedler U and Rudolph R, WO01/04144); green fluorescent protein (GFP) (Peelle B et al (2001) Chem. Biol. 8:521-534); human cytotoxic T lymphocyte-associated antigen 4 (CTLA-4) (Hufton SE et al (2000) FEBS Lett. 475:225-231; Irving RA et al (2001) J. Immunol. Meth. 248:31-45); protease inhibitors, such as Knottin proteins (Wentzel A et al (2001) J. Bacteriol. 183:7273-7284; Baggio R et al (2002) J. Mol. Recognit. 15:126-134) and Kunitz domains (Roberts BL et al (1992) Gene 121:9-15; Dennis MS and Lazarus RA (1994) J. Biol. Chem. 269:22137-22144); PDZ domains (Schneider S et al (1999) Nat. Biotechnol. 17:170-175); peptide aptamers, such as thioredoxin (Lu Z et al (1995) Biotechnology 13:366-372; Klevenz B et al (2002) Cell. Mol. Life Sci. 59:1993-1998); staphylococcal nuclease (Norman TC et al (1999) Science 285:591-595); tendamistats (McConell SJ and Hoess RH (1995) J. Mol. Biol. 250:460-479; Li R et al (2003) Protein Eng. 16:65-72); trinectins based on the fibronectin type III domain (Koide A et al (1998) J. Mol. Biol. 284:1141-1151; Xu L et al (2002) Chem. Biol. 9:933-942); and zinc fingers (Bianchi E et al (1995) J. Mol. Biol. 247:154-160; Klug A (1999) J. Mol. Biol. 293:215-218; Segal DJ et al (2003) Biochemistry 42:2137-2148).

The above mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), mRNA display (Roberts RW and Szostak JW (1997) Proc. Natl. Acad. Sci. U.S.A. 94:12297-12302), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the methods of the above aspects, an affinity ligand may be used, which is a non-immunoglobulin affinity ligand derived from any of the protein scaffolds listed above, or an oligonucleotide molecule.

In some embodiments of the methods of the above aspects, an affinity ligand capable of selective interaction with the GPX2 protein is detectable and/or quantifiable. The detection and/or quantification of such an affinity ligand may be accomplished in any way known to the skilled person for detection and/or quantification of binding reagents in assays based on biological interactions. Thus, any affinity ligand, as described in the previous section, may be used quantitatively or qualitatively to detect the presence of the GPX2 protein. These "primary" affinity ligands may be labeled themselves with various markers or are in turn detected by secondary, labeled affinity ligands to allow detection, visualization and/or quantification. This can be accomplished using any one or more of a multitude of labels, which can be conjugated to the affinity ligand capable of interaction with GPX2 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g. horseradish peroxidase, alkaline phosphatase, beta-lactamase), radioisotopes (e.g. ³H, ¹⁴C, ³²P, ³⁵S or ¹²⁵I) and particles (e.g. gold). The different types of labels can be conjugated to an affinity ligand using various chemistries, e.g. the amine reaction or the thiol reaction. However, other reactive groups than amines and thiols can be used, e.g. aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its GPX2 protein target may involve visual techniques, such as light microscopy or immunofluoresence microscopy. Other methods may involve the detection via flow cytometry or luminometry.

As explained above, detection and/or quantification of GPX2 protein in a subject may be accomplished by removing a biological sample from the subject, such as a tissue sample (biopsy), for example from colorectal tissue or urothelial tissue, blood sample, cerebral fluid, urine or stool. Alternatively, the biological sample, such as a biopsy, is an earlier obtained sample. If using an earlier obtained sample, no steps of the any of the embodiments of the methods of the above aspects are practiced on the human or animal body. The affinity ligand is applied to the biological sample for detection and/or quantification of the GPX2 marker protein. This procedure enables not only detection of GPX2 protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light of a specific wavelength. The presence of a luminescently tagged affinity ligand may be detected and/or quantified by luminescence developed during a chemical reaction. Detection of an enzymatic reaction is due to a color shift in the sample arising from chemical reaction. Those of skill in the art are aware that a variety of different protocols can be modified in order for proper detection and/or quantification.

In the method according to the invention, a biological sample may be immobilized onto a solid phase support or carrier, such as nitrocellulose or any other solid support matrix capable of immobilizing any GPX2 protein present in the biological sample applied to it. Some well-known solid state support materials useful in the present invention include glass, carbohydrate (e.g. Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand specific to GPX2 may be applied, e.g. according to Examples, sections 2 and/or 3, of the present disclosure. If the primary affinity ligand is not labeled in itself, the supporting matrix is washed with various buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art will be able to determine operative and optimal assay conditions for each determination by routine experimentation.

A method to detect and/or quantify the GPX2 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the GPX2 protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the current invention are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize GPX2 protein by detection of radioactivity *in vivo* or *in vitro.* Radionuclear scanning with e.g. gamma camera, magnetic resonance spectroscopy or emission tomography function for detection *in vivo* and *in vitro,* while gamma/beta counters, scintillation counters and radiographies are also used *in vitro.*

In embodiments of the methods of the aspects above, the subject is a human.

In embodiments of the methods of the aspects above, the subject is having a cancer.

In embodiments of the methods of the aspects above, all steps are preformed *in vitro.*

A further aspect of the present invention provides a kit for carrying out the methods according to the above aspects, which kit comprises:
a) a quantifiable affinity ligand capable of selective interaction with a GPX2 protein; and
b) reagents necessary for quantifying the amount of the affinity ligand.

The various components of the kit according to the invention are selected and specified as described above in connection with the method aspects of the present invention.

Thus, the kit according to the invention comprises an affinity ligand against GPX2, as well as other means that help to quantify the specific and/or selective affinity ligand after it has bound specifically and/or selectively to GPX2. For example, the kit may contain a secondary affinity ligand for detecting and/or quantifying a complex formed by any GPX2 protein and the affinity ligand capable of selective interaction with a GPX2 protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, and substances such as reaction arresters that are commonly used in immunoassay reagent kits.

The kit may also advantageously comprise a reference sample for provision of, or yielding, the reference value to be used for comparison with the sample value. Such a reference sample may for example be constituted by a sample of tissue having a predetermined amount of GPX2 protein, which may then be used by the person of skill in the art of pathology to determine the GPX2 expression status in the sample being studied, by ocular or automated comparison of expression levels in the reference sample and the subject sample.

The wording "reference sample for provision of the reference value" is to be interpreted broadly in the context of the kit aspect. The reference sample may contain an amount of GPX2 protein actually corresponding to the reference value, but it may also contain an amount of GPX2 protein corresponding to a value being higher than the reference value. In the latter case, the "high" value may be used by a person performing the method as an upper reference for assessing, e.g. the appearance of, a lower reference value. The skilled person understands how to do such an assessment.

A sample comprising essentially no GPX2 protein may correspond to a very poor prognosis for a subject having a cancer. One way to establish if a sample essentially lacks GPX2 protein is to compare it with a reference sample essentially lacking GPX2 protein. Such comparison may for example be visual. Consequently, in an embodiment of the kit aspect, the reference sample is a tissue sample with essentially no GPX2 protein present. "Essentially no GPX2 protein present" refers to an amount of GPX2 protein, which is so small that it is not detectable, during normal circumstances, by a person using the kit.

Also, the sample value may be compared to a reference value corresponding to a predetermined amount of GPX2 protein comprised in a reference sample. Consequently, in embodiments of the kit aspect, the reference sample may comprise an amount of GPX2 protein corresponding to a cytoplasmic fraction of 50 % GPX2 positive cells or lower, such as 40 % GPX2 positive cells or lower, such as 30 % GPX2 positive cells or lower, such as 25 % GPX2 positive cells or lower, such as 20 % GPX2 positive cells or lower, such as 15 % GPX2 positive cells or lower, such as 10 % GPX2 positive cells or lower, such as 5 % GPX2 positive cells or lower, such as 2 % GPX2 positive cells or lower. Alternatively, or as a complement, the reference sample may comprise an amount of GPX2 protein corresponding to a moderate GPX2 expression cytoplasmic intensity or lower, such as a faint GPX2 expression cytoplasmic intensity or lower, such as no GPX2 expression cytoplasmic intensity. The determination of a "cytoplasmic fraction" or a "cytoplasmic intensity" for the reference sample may for example be performed as described below in Examples, section 3 and section 4, definition of "cytoplasmic fraction" or "cytoplasmic intensity". Also, the provision of cytoplasmic fraction values or cytoplasmic intensity values is discussed above in connection with the method aspect.

Further, the reference sample may contain a high amount of GPX2 protein, e.g. for establishing the appearance of a sample with a high amount of GPX2 protein after histochemical staining. Such reference sample may be a sample comprising tissue expressing a high amount of GPX2, such as normal epithelial tissue from urine bladder. Referring to "cytoplasmic fraction", the reference sample may provide examples of cells with moderate or strong immunoreactivity (positive staining) and/or an example of a sample with more than 25 % positive cells. Referring to "cytoplasmic intensity", the reference sample may provide an example of a strong immunoreactivity.

Also referring to "cytoplasmic intensity", the reference sample may contain an amount of GPX2 protein corresponding to a strong immunoreactivity. With the knowledge of the appearance of a sample with strong immunoreactivity (= distinct and strong immunoreactivity), the skilled artisan may assess reference values representing the categories presented in Examples, section 3, below, i.e. absent (=no immunoreactivity), weak (= faint immunoreactivity), moderate (= medium immunoreactivity) and strong (= distinct and strong immunoreactivity). Further, referring to "cytoplasmic fraction", the reference sample may contain an amount of GPX2 protein corresponding to, e.g., more than 75 % positive cells. With the knowledge of the appearance of a sample with more than 75 % positive cells, the skilled artisan may assess reference values representing "cytoplasmic fraction" having e.g. a lower percentage of positive cells.

In embodiments of the kit aspect, the reference sample may be a tissue sample, such as a tissue sample adapted to ocular or microscopic evaluation. As an example, the tissue reference sample may also be adapted to staining with affinity ligands, such as antibodies, for a GPX2 protein.

In another embodiment of the kit aspect, the reference sample may comprise cancer cell lines expressing a predetermined, or controlled, amount of GPX2 protein. The person skilled in the art understands how to provide such cell lines, for example guided by the disclosure of Rhodes et al. (2006) The biomedical scientist, p 515-520.

As a further aspect of the present invention, there is provided the use of a GPX2 protein as a prognostic marker. Also provided is the use of a GPX2 protein as a prognostic marker for a cancer.

As a related aspect of the invention, there is provided the use of a GPX2 protein, or an antigenically active fragment thereof, in the manufacture of a prognostic agent for prognosticating the outcome of a cancer. An antigenically active fragment of a GPX2 protein is a fragment of sufficient size to be useful for the generation of an affinity ligand, e.g. an antibody, which will interact with a GPX2 protein comprising the fragment.

In embodiments of these use aspects of the invention, the GPX2 protein may, as a non-limiting example, have an amino acid sequence which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the GPX2 protein may have an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

Referring to the discussion in connection with the method aspects above, in embodiments of the use aspects, the cancer may be a malignant epithelial tumor, such as a malignant tumor in the gastrointestinal tract including accessory organs or a urothelial cancer. Further, the cancer in question may be a malignant tumor in the gastrointestinal tract, such as an adenocarcinoma in the gastrointestinal tract. Also, the cancer in question may be a stomach cancer, a pancreatic cancer, a liver cancer or a colorectal cancer, such as colorectal cancer in Dukes' stage A, B or C. As an example, the cancer in question may be selected from the group consisting of stomach cancer, pancreatic cancer, liver cancer, colorectal cancer and urothelial cancer. Further, the cancer in question may be selected from the group consisting of stomach cancer, pancreatic cancer, liver cancer and colorectal cancer. Alternatively, the cancer in question may be selected from the group consisting of urothelial cancer and colorectal cancer.

In a further aspect, the present invention provides a GPX2 protein for establishing a prognosis for a mammalian subject having or suspected of having a cancer. In the context of the present disclosure, "establishing a prognosis " refers to establishing a specific prognosis or a prognosis interval. In embodiments of this aspect, the amino acid sequence of the GPX2 protein may, as a non-limiting example, comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the GPX2 protein may have an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

As a further aspect thereof, the present invention provides an affinity ligand capable of selective interaction with a GPX2 protein, which is an antibody or a fragment or a derivative thereof. Such an antibody, or fragment or derivative thereof, may for example be one that is obtainable by a process comprising a step of immunizing an animal with a protein whose amino acid sequence comprises the sequence SEQ ID NO:1. Processes for the production of antibodies or fragments or derivatives thereof against a given target are known in the art, and may be applied in connection with this aspect of the present invention. Any of those variants of the GPX2 protein (SEQ ID NO:2) or the antigenically active fragment thereof (SEQ ID NO:1) that are discussed above may, of course, be used in such a process for generating an antibody or a fragment or derivative thereof. The present invention also provides the above affinity ligand for establishing a prognosis for a mammalian subject having a cancer.

As a further aspect thereof, the present invention provides use of the affinity ligand according to the invention as a prognostic agent. A preferred embodiment of this use is use of the affinity ligand as a prognostic agent for the prognosis of a cancer. As a related aspect thereof, the present invention provides use of the affinity ligand in the manufacture of a prognostic agent for the prognosis of a cancer.

Again referring to the discussion in connection with the method aspects above, in embodiments of the aspects relating to a GPX2 protein or an affinity ligand above, the cancer may be a malignant epithelial tumor, such as a malignant tumor in the gastrointestinal tract including accessory organs or a urothelial cancer. Further, the cancer in question may be a malignant tumor in the gastrointestinal tract, such as an adenocarcinoma in the gastrointestinal tract. Also, the cancer in question may be a stomach cancer, a pancreatic cancer, a liver cancer or a colorectal cancer, such as colorectal cancer in Dukes' stage A, B or C. As an example, the cancer in question may be selected from the group consisting of stomach cancer, pancreatic cancer, liver cancer, colorectal cancer and urothelial cancer. Further, the cancer in question may be selected from the group consisting of stomach cancer, pancreatic cancer, liver cancer and colorectal cancer. Alternatively, the cancer in question may be selected from the group consisting of urothelial cancer and colorectal cancer.

### Brief description of the figures

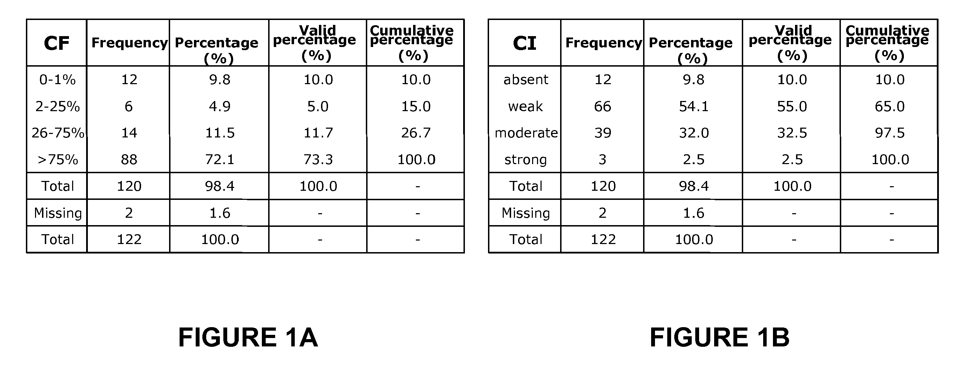

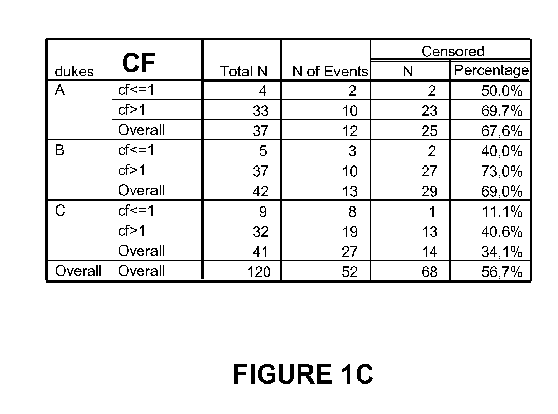

Figure 1 shows the level of immunohistochemical staining of GPX2 in 122 patients diagnosed with colorectal carcinomas. The subjects were scored for GPX2 expression. Figure 1A shows level of cytoplasmic fraction (CF). Figure 1B shows level of cytoplasmic intensity (CI). In figure 1C, subjects were first separated into Dukes' stages and than divided according to CF (N = no of subjects).
Figure 2 shows the results of a survival analysis based on immunohistochemical staining of 120 subjects diagnosed with colo-rectal carcinomas. Figure 2A shows level of cytoplasmic fraction. The solid line represents >25% positive cells (> 1), and the dotted line represents <=25% positive cells (<= 1). Figure 2B shows level of cytoplasmic intensity. The solid line represents a faint, moderate or strong immunoreactivity (> 0), and the dotted line represents no immunoreactivity (= 0).

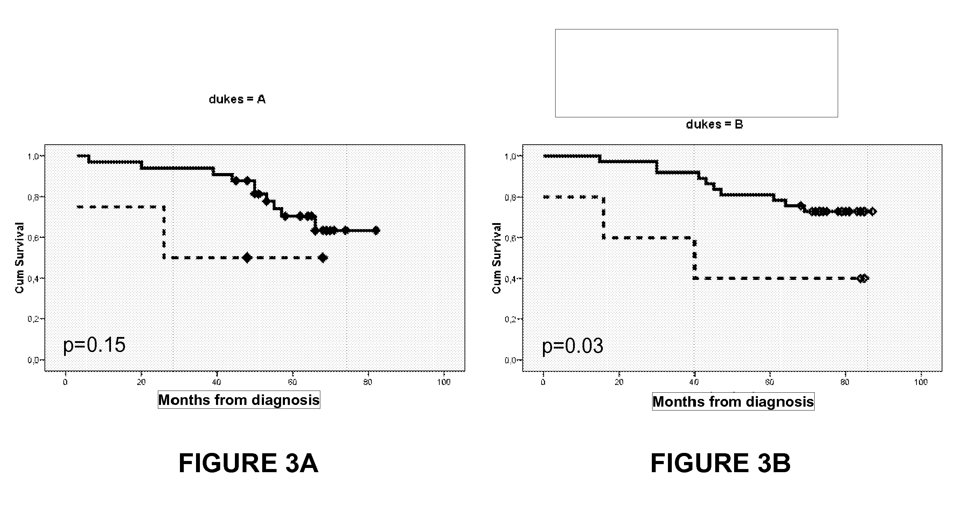

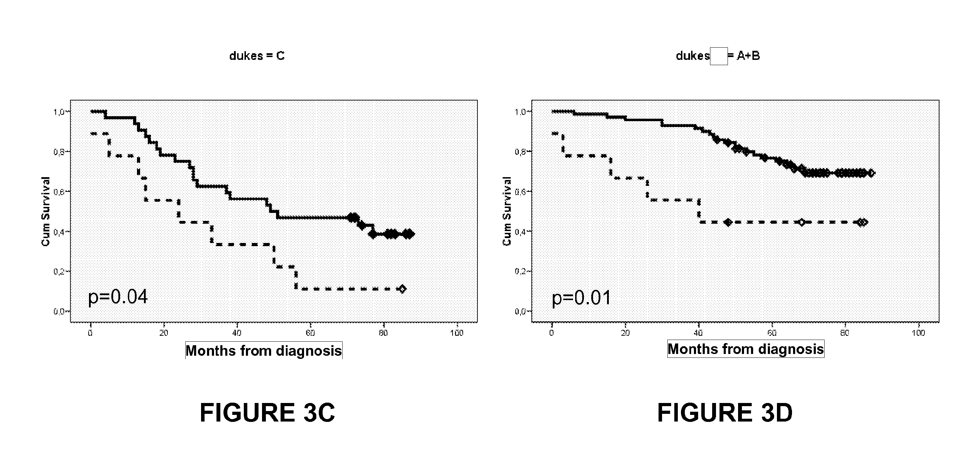

Figure 3 shows the results of a survival analysis based on immunohistochemical staining of 120 subjects diagnosed with colorectal carcinomas. The subjects were scored for high or low GPX2 cytoplasmic fraction. The solid line represents >25% positive cells, and the dotted line represents <=25% positive cells. The subjects where then further divided into three groups according to Dukes' stages: stage A (3A), stage B (3B), stage C (3C) and stage A and B (3D).

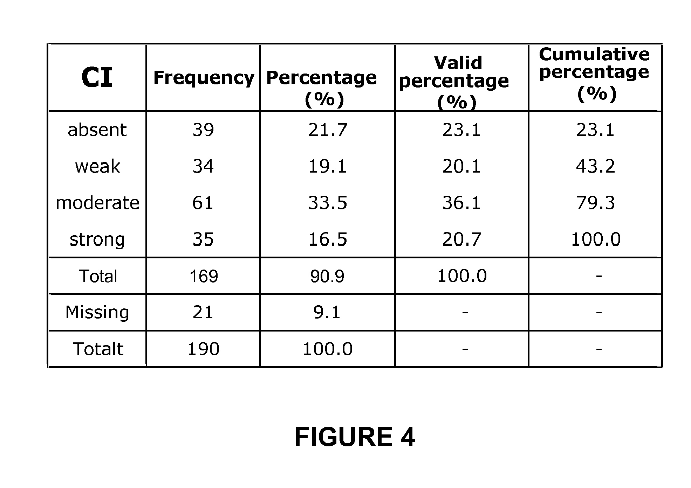

Figure 4 shows the level of immunohistochemical staining of GPX2 in 190 subjects diagnosed with urothelial carcinomas. Subjects were scored for level of cytoplasmic intensity.

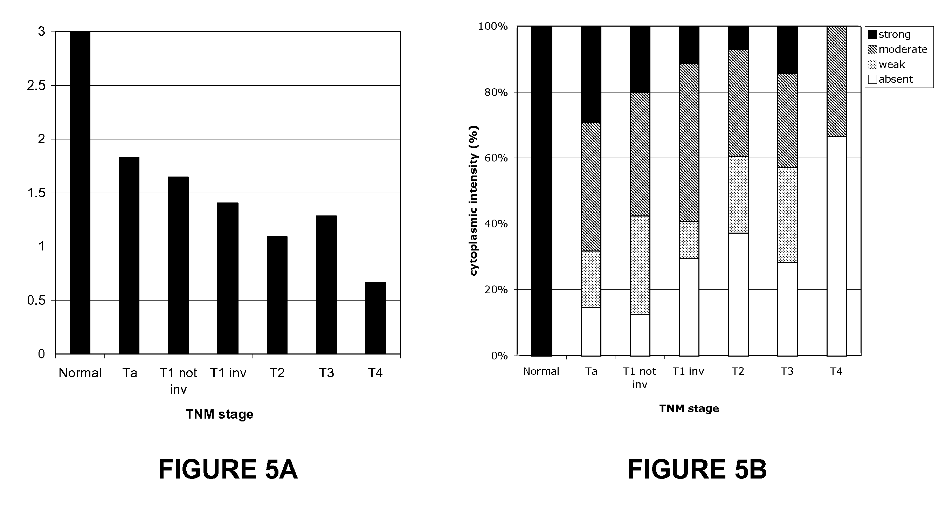

Figure 5 shows the level of immunohistochemical staining of GPX2 in 169 subjects diagnosed with urothelial carcinomas. Subjects were scored for level of cytoplasmic intensity and an average was calculated based on TNM stage, figure 5A. In figure 5A, the values 0,1, 2 and 3 corresponds to absent, weak, moderate and strong cytoplasmic intensity, respectively. Figure 5B shows the relative abundance of different cytoplasmic intensity categories for each stage.
Figure 6 shows the level of immunohistochemical staining of GPX2 in 20 primary tumors and 20 metastases from 20 patients diagnosed with urothelial carcinomas. Subjects were scored for level of cytoplasmic intensity and then an average was calculated, figure 6A. In figure 6A, the values 0,1, 2 and 3 corresponds to absent, weak, moderate and strong cytoplasmic intensity, respectively. Figure 6B shows the relative abundance of different cytoplasmic intensity categories.

### Examples

### Generation of mono-specific antibodies against GPX2 and use thereof to detect GPX2 in normal and cancerous samples

### 1) Generation of antigen

### a. Materials and methods

A suitable fragment of the target protein encoded by the EnsEMBL Gene ID ENSG00000176153 was designed using bioinformatic tools with the human genome sequence as template (Lindskog M et al (2005) Biotechniques 38:723-727, EnsEMBL, www.ensembl.org). A fragment consisting of 89 amino acids corresponding to amino acids 63-150 (SEQ ID NO:1) of the protein GPX2 (SEQ ID NO:2; EnsEMBL entry no. ENSP00000374265) was designed. A polynucleotide encoding the target protein, which polynucleotide contained nucleotides 187-543 of the long GPX2 gene transcript (SEQ ID NO:3; EnsEMBL entry no. ENST00000389614), was isolated by a Superscript™ One-Step RT-PCR amplification kit with Platinum® Taq (Invitrogen) and a human total RNA pool panel as template (Human Total RNA Panel IV, BD Biosciences Clontech). Flanking restriction sites Notl and Ascl were introduced into the fragment through the PCR amplification primers, to allow in-frame cloning into the expression vector (forward primer: GTCCTTGGCTTCCCTTGC, reverse primer: GGCCACATCTGAGCGGC). Then, the downstream primer was biotinylated to allow solid-phase cloning as previously described, and the resulting biotinylated PCR product was immobilized onto Dynabeads M280 Streptavidin (Invitrogen Dynal Biotech) (Larsson M et al (2000) J. Biotechnol. 80:143-157). The fragment was released from the solid support by Notl-Ascl digestion (New England Biolabs), ligated into the pAff8c vector (Larsson M *et al, supra*) in frame with a dual affinity tag consisting of a hexahistidyl tag for immobilized metal ion chromatography (IMAC) purification and an immunopotentiating albumin binding protein (ABP) from streptococcal protein G (Sjölander A et al (1997) J. Immunol. Methods 201:115-123; Ståhl S et al (1999) Encyclopedia of Bioprocess Technology: Fermentation, Biocatalysis and Bioseparation (Fleckinger MC and Drew SW, eds) John Wiley and Sons Inc., New York, pp 49-63), and transformed into *E. coli* BL21 (DE3) cells (Novagen). The sequences of the clones were verified by dye-terminator cycle sequencing of plasmid DNA amplified using TempliPhi DNA sequencing amplification kit (GE Healthcare, Uppsala, Sweden) according to the manufacturer's recommendations.

BL21 (DE3) cells harboring the expression vector were inoculated in 100 ml 30 g/I tryptic soy broth (Merck KGaA) supplemented with 5 g/I yeast extract (Merck KGaA) and 50 mg/l kanamycin (Sigma-Aldrich) by addition of 1 ml of an overnight culture in the same culture medium. The cell culture was incubated in a 1 liter shake flask at 37 °C and 150 rpm until the optical density at 600 nm reached 0.5-1.5. Protein expression was then induced by addition of isopropyl-β-D-thiogalactopyranoside (Apollo Scientific) to a final concentration of 1 mM, and the incubation was continued overnight at 25 °C and 150 rpm. The cells were harvested by centrifugation at 2400 *g*, and the pellet was re-suspended in 5 ml lysis buffer (7 M guanidine hydrochloride, 47 mM Na₂HPO₄, 2.65 mM NaH₂PO₄, 10 mM Tris-HCI, 100 mM NaCl, 20 mM β-mercaptoethanol; pH = 8.0) and incubated for 2 hours at 37 °C and 150 rpm. After centrifugation at 35300 g, the supernatant containing the denatured and solubilized gene products was collected.

The His₆-tagged fusion protein was purified by immobilized metal ion affinity chromatography (IMAC) on columns with 1 ml Talon® metal (Co²⁺) affinity resin (BD Biosciences Clontech) using an automated protein purification procedure (Steen J et al (2006) Protein Expr. Purif. 46:173-178) on an ASPEC XL4™ (Gilson). The resin was equilibrated with 20 ml denaturing washing buffer (6 M guanidine hydrochloride, 46.6 mM Na₂HPO₄, 3.4 mM NaH₂PO₄, 300 mM NaCl, pH 8.0-8.2). The resin was then washed with a minimum of 31.5 ml washing buffer prior to elution in 2.5 ml elution buffer (6 M urea, 50 mM NaH₂PO₄, 100 mM NaCl, 30 mM acetic acid, 70 mM Na-acetate, pH 5.0). The eluted material was fractioned in three pools of 500, 700 and 1300 µl. The 700 µl fraction, containing the antigen, and the pooled 500 and 1300 µl fractions were stored for further use.

The antigen fraction was diluted to a final concentration of 1 M urea with phosphate buffered saline (PBS; 1.9 mM NaH₂PO₄, 8.1 mM Na₂HPO₄, 154 mM NaCl) followed by a concentration step to increase the protein concentration using Vivapore 10/20 ml concentrator with molecular weight cut off at 7500 Da (Vivascience AG). The protein concentration was determined using a bicinchoninic acid (BCA) micro assay protocol (Pierce) with a bovine serum albumin standard according to the manufacturer's recommendations. The protein quality was analyzed on a Bioanalyzer instrument using the Protein 50 or 200 assay (Agilent Technologies).

### b) Results

A gene fragment corresponding to nucleotides 187-543 of the long transcript (SEQ ID NO:3) of the GPX2 gene and encoding a peptide (SEQ ID NO:1) consisting of amino acids 63 to 150 of the target protein GPX2 (SEQ ID NO:2) was successfully isolated by RT-PCR from a human RNA pool using primers specific for the protein fragment. The 89 amino acid fragment (SEQ ID NO:1) of the target protein (SEQ ID NO:2) was designed to lack transmembrane regions to ensure efficient expression in *E. coli,* and to lack any signal peptide, since those are cleaved off in the mature protein. In addition, the protein fragment was designed to consist of a unique sequence with low homology with other human proteins, to minimize cross reactivity of generated affinity reagents, and to be of a suitable size to allow the formation of conformational epitopes and still allow efficient cloning and expression in bacterial systems.

A clone encoding the correct amino acid sequence was identified, and, upon expression in *E. coli,* a single protein of the correct size was produced and subsequently purified using immobilized metal ion chromatography. After dilution of the eluted sample to a final concentration of 1 M urea and concentration of the sample to 1 ml, the concentration of the protein fragment was determined to be 10.5 mg/ml and was 100 % pure according to purity analysis.

### 2) Generation of antibodies

### a) Materials and methods

The purified GPX2 fragment as obtained above was used as antigen to immunize a rabbit in accordance with the national guidelines (Swedish permit no. A 84-02). The rabbit was immunized intramuscularly with 200 µg of antigen in Freund's complete adjuvant as the primary immunization, and boosted three times in four-week intervals with 100 µg antigen in Freund's incomplete adjuvant.

Antiserum from the immunized animal was purified by a three-step immunoaffinity based protocol (Agaton C et al (2004) J. Chromatogr. A 1043:33-40; Nilsson P et al (2005) Proteomics 5:4327-4337). In the first step, 7 ml of total antiserum was buffered with 10x PBS to a final concentration of 1x PBS (1.9 mM NaH₂PO₄, 8.1 mM Na₂HPO₄, 154 mM NaCl), filtered using a 0.45 µm pore-size filter (Acrodisc®, Life Science) and applied to a an affinity column containing 5 ml N-hydroxysuccinimide-activated Sepharose™ 4 Fast Flow (GE Healthcare) coupled to the dual affinity tag protein His₆-ABP (a hexahistidyl tag and an albumin binding protein tag) expressed from the pAff8c vector and purified in the same way as described above for the antigen protein fragment. In the second step, the flow-through, depleted of antibodies against the dual affinity tag His₆-ABP, was loaded at a flow rate of 0.5 ml/min on a 1 ml Hi-Trap NHS-activated HP column (GE Healthcare) coupled to the GPX2 protein fragment used as antigen for immunization (SEQ ID NO:1). The His₆-ABP protein and the protein fragment antigen had been coupled to the NHS activated matrix as recommended by the manufacturer. Unbound material was washed away with 1 x PBST (1 x PBS, 0.1 % Tween20, pH 7.25), and captured antibodies were eluted using a low pH glycine buffer (0.2 M glycine, 1 mM EGTA, pH 2.5). The eluted antibody fraction was collected automatically, and loaded onto two 5 ml HiTrap™ desalting columns (GE Healthcare) connected in series for efficient buffer exchange in the third step. The second and third purification steps were run on the ÄKTAxpress™ platform (GE Healthcare). The antigen selective (mono-specific) antibodies (msAbs) were eluted with PBS buffer, supplemented with glycerol and NaN₃ to final concentrations of 50 % and 0.02 %, respectively, for long term storage at -20 °C (Nilsson P et al (2005) Proteomics 5:4327-4337).

The specificity and selectivity of the affinity purified antibody fraction were analyzed by binding analysis against the antigen itself and against 94 other human protein fragments in a protein array set-up (Nilsson P et al (2005) Proteomics 5:4327-4337). The protein fragments were diluted to 40 µg/ml in 0.1 M urea and 1x PBS (pH 7.4) and 50 µl of each was transferred to the wells of a 96-well spotting plate. The protein fragments were spotted and immobilized onto epoxy slides (SuperEpoxy, TeleChem) using a pin-and-ring arrayer (Affymetrix 427). The slide was washed in 1x PBS (5 min) and the surface was then blocked (SuperBlock®, Pierce) for 30 minutes. An adhesive 16-well silicone mask (Schleicher & Schuell) was applied to the glass before the mono-specific antibodies were added (diluted 1:2000 in 1x PBST to appr. 50 ng/ml) and incubated on a shaker for 60 min. Affinity tag-specific IgY antibodies were co-incubated with the mono-specific antibodies in order to quantify the amount of protein in each spot. The slide was washed with 1x PBST and 1x PBS twice for 10 min each. Secondary antibodies (goat anti-rabbit antibody conjugated with Alexa 647 and goat anti-chicken antibody conjugated with Alexa 555, Molecular Probes) were diluted 1:60000 to 30 ng/ml in 1x PBST and incubated for 60 min. After the same washing procedure as for the first incubation, the slide was spinned dry and scanned, (G2565BA array scanner, Agilent) and images were quantified using image analysis software (GenePix 5.1, Axon Instruments).

### b) Results

The quality of polyclonal antibody preparations has proven to be dependent on the degree of stringency in the antibody purifications, and it has previously been shown that depletion of antibodies directed against epitopes not originated from the target protein is necessary to avoid cross-reactivity to other proteins and background binding (Agaton C et al (2004) J. Chromatogr. A 1043:33-40).

Thus, a protein microarray analysis was performed to ensure that mono-specific polyclonal antibodies of high specificity had been generated by depletion of antibodies directed against the His₆-tag as well as of antibodies against the ABP-tag. This was followed by affinity capture of antigen selective antibodies on an affinity column with immobilized antigen.

To quantify the amount of protein in each spot of the protein array, a two-color dye labeling system was used, with a combination of primary and secondary antibodies. Tag-specific IgY antibodies generated in hen were detected with a secondary goat anti-hen antibody labeled with Alexa 555 fluorescent dye. The specific binding of the rabbit msAb to its antigen on the array was detected with a fluorescently Alexa 647 labeled goat anti-rabbit antibody. Each protein fragment was spotted in duplicates. The protein array analysis shows that the affinity purified mono-specific antibody against GPX2 is highly selective to the correct protein fragment and shows a very low background to all other protein fragments analyzed on the array.

### 3) Tissue profiling by immunohistochemistry

### a) Material and Methods

In total, 576 paraffin cores containing human tissues were analyzed using the mono-specific antibody sample. All tissues used as donor blocks for tissue microarray (TMA) production were selected from the archives at the Department of Pathology, University Hospital, Uppsala, in agreement with approval from the local ethical committee. Corresponding tissue sections were examined to determine diagnosis and to select representative areas in donor blocks. Normal tissue was defined as microscopically normal (non-neoplastic) and was most often selected from specimens collected from the vicinity of surgically removed tumors. All tissues were formalin fixated, paraffin embedded, and sectioned for diagnostic purposes.

The TMA production was performed essentially as previously described (Kononen J et al (1998) Nature Med. 4:844-847; Kallioniemi OP et al (2001) Hum. Mol. Genet. 10:657-662). Briefly, a hole was made in the recipient TMA block. A cylindrical core tissue sample from the donor block was acquired and deposited in the recipient TMA block. This was repeated in an automated tissue arrayer from Beecher Instrument (ATA-27, Beecher Instruments, Sun Prairie, CA, USA) until a complete TMA design was produced. TMA recipient blocks were baked at 42 °C for 2 h prior to sectioning.

The design of TMA:s was focused on obtaining samples from a large range of representative normal tissues, and on including representative cancer tissues. This has previously been described in detail in Kampf C et al (2004) Clin. Proteomics 1:285-300. In brief, samples from 48 normal tissues and from 20 of the most common cancer types affecting humans were selected. In total, eight different designs of TMA blocks, each containing 72 cores of tissue with 1 mm diameter, were produced. Two of the TMA:s represented normal tissues, corresponding to 48 different normal tissues in triplicates from different individuals. The remaining 6 TMA:s represented cancer tissue from 20 different types of cancer. For 17 of the 20 cancer types, 12 individually different tumors were sampled, and for the remaining 3 cancer types, 4 individually different tumors were sampled, all in duplicates from the same tumor. The TMA blocks were sectioned with 4 µm thickness using a waterfall microtome (Leica), and placed onto SuperFrost® (Roche Applied Science) glass slides for immunohistochemical analysis.

Automated immunohistochemistry was performed as previously described (Kampf C et al (2004) Clin. Proteomics 1:285-300). In brief, the glass slides were incubated for 45 min in 60 °C, de-paraffinized in xylene (2 x 15 min) and hydrated in graded alcohols. For antigen retrieval, slides were immersed in TRS (Target Retrieval Solution, pH 6.0, DakoCytomation) and boiled for 4 min at 125 °C in a Decloaking chamber® (Biocare Medical). Slides were placed in the Autostainer® (DakoCytomation) and endogenous peroxidase was initially blocked with H₂O₂ (DakoCytomation). The primary antibody generated in section 2 and goat anti-rabbit peroxidase conjugated Envision® were incubated for 30 min each at room temperature. Between all steps, slides were rinsed in wash buffer (DakoCytomation). Finally, diaminobenzidine (DakoCytomation) was used as chromogen and Harris hematoxylin (Sigma-Aldrich) was used for counterstaining. The slides were mounted with Pertex® (Histolab).

All immunohistochemically stained sections from the eight different TMA:s were scanned using a ScanScope T2 automated slide-scanning systems (Aperio Technologies). In order to represent the total content of the eight TMA:s, 576 digital images were generated. Scanning was performed at 20 times magnification. Digital images were separated and extracted as individual tagged image file format (TIFF) files for storage of original data. In order to be able to handle the images in a web-based annotation system, the individual images were compressed from TIFF format into JPEG format. All images of immunohistochemically stained tissue were manually evaluated under the microscope and annotated by a board of certified pathologist or by specially educated personnel (followed by verification of a pathologist). Annotation of each different normal and cancer tissue was performed using a simplified scheme for classification of immunohistochemical outcome. Each tissue was examined for representativity and immunoreactivity. The different tissue specific cell types included in each normal tissue type were annotated. For each cancer, tumor cells and stroma were annotated. Basic annotation parameters included an evaluation of i) subcellular localization (nuclear and/or cytoplasmic/ membranous), ii) staining intensity and iii) fraction of stained cells. Staining intensity was subjectively evaluated in accordance to standards used in clinical histo-pathological diagnostics and outcome was classified as: absent = no immunoreactivity, weak = faint immunoreactivity, moderate = medium immunoreactivity or strong = distinct and strong immunoreactivity. The fraction of stained cells was estimated and classified as < 2 %, 2-25 %, 26-75 % or > 75 % immunoreactive cells of the relevant cell population. Based on both the intensity and fraction of immunoreactive cells, a "staining score" was given for each tissue sample: 0 = negative, 1 = weak, 2 = moderate and 3 = strong. N.R. means that no representative tissues were present.

### b) Results

The results from tissue profiling with the mono-specific antibody generated towards a recombinant protein fragment of the human target protein GPX2 in section 2 showed a particular immunoreactivity in several normal and cancer tissues (Tables 1-2).

Table 1 shows the GPX2 protein expression pattern in normal human tissues. Using immunohistochemistry and TMA technology, 144 spots (1 mm in diameter) representing 48 different types of normal tissue were screened for expression of GPX2. GPX2 expression was mainly detected in the cytoplasm epithelial cells, such as glandular or surface epithelial cells. Strong expression was found in the urinary bladder. Moderate levels of expression were detected in the appendix, gall bladder, heart muscle, rectum and stomach.

| **Table 1:** Expression pattern of GPX2 in normal tissues | | |
|---|---|---|
| **Tissue type** | **Cell type** | **Staining score** |
| **Adrenal gland** | cortical cells | 0 |
| | medullar cells | 0 |
| **Appendix** | glandular cells | 2 |
| | lymphoid tissue | N.R. |
| **Bone marrow** | bone marrow poetic cells | 0 |
| **Breast** | glandular cells | 0 |
| **Bronchus** | surface epithelial cells | 1 |
| **Cerebellum** | cells in granular layer | 0 |
| | cells in molecular layer | 0 |
| | purkinje cells | 1 |
| **Cerebral cortex** | neuronal cells | 0 |
| | non-neuronal cells | 0 |
| **Cervix, uterine** | glandular cells | 0 |
| | surface epithelial cells (squamous) | 0 |
| **Colon** | glandular cells | 1 |
| **Duodenum** | glandular cells | 1 |
| **Endometrium 1** | cells in endometrial stroma/ECM | 0 |
| | cells in myometrium/ECM | 0 |
| | glandular cells | 0 |
| **Endometrium 2** | cells in endometrial stroma/ECM | 0 |
| | cells in myometrium/ECM | 0 |
| | glandular cells | 0 |
| **Epididymis** | glandular cells | 1 |
| **Esophagus** | surface epithelial cells | 0 |
| **Fallopian tube** | glandular cells | 0 |
| **Gall bladder** | glandular cells | 2 |
| **Heart muscle** | myocytes | 2 |
| **Hippocampus** | neuronal cells | 1 |
| | non-neuronal cells | 0 |
| **Kidney** | cells in glomeruli | 0 |
| | cells in tubuli | 1 |
| **Lateral ventricle** | neuronal cells | 1 |
| | non-neuronal cells | 0 |
| **Liver** | bile duct cells | 1 |
| | hepatocytes | 0 |
| **Lung** | alveolar cells | 0 |
| | macrophages | 1 |
| **Lymph node** | follicle cells (cortex) | 0 |
| | non-follicle cells (paracortex) | 0 |
| **Nasopharynx** | surface epithelial cells | 1 |
| **Oral mucosa** | surface epithelial cells | 0 |
| **Ovary** | follicle cells | 0 |
| | ovarian stromal cells | 0 |
| **Pancreas** | exocrine pancreas | 0 |
| | islet cells | 0 |
| **Parathyroid gland** | glandular cells | 1 |
| **Placenta** | decidual cells | 0 |
| | trophoblastic cells | 0 |
| **Prostate** | glandular cells | 0 |
| **Rectum** | glandular cells | 2 |
| **Salivary gland** | glandular cells | 0 |
| **Seminal vescicle** | glandular cells | 0 |
| **Skeletal muscle** | myocytes | 0 |
| **Skin** | adnexal cells | 0 |
| | epidermal cells | 0 |
| **Small intestine** | glandular cells | 1 |
| **Smooth muscle** | smooth muscle cells | 0 |
| **Soft tissue 1** | mesenchymal cells | 0 |
| **Soft tissue 2** | mesenchymal cells | 0 |
| **Spleen** | cells in red pulp | 0 |
| | cells in white pulp | 0 |
| **Stomach 1** | glandular cells | 1 |
| **Stomach 2** | glandular cells | 2 |
| **Testis** | cells in ductus seminiferus | 0 |
| | leydig cells | 0 |
| **Thyroid gland** | glandular cells | 0 |
| **Tonsil** | follicle cells (cortex) | 0 |
| | non-follicle cells (paracortex) | 0 |
| | surface epithelial cells | N.R. |
| **Urinary bladder** | surface epithelial cells | 3 |
| **Vagina** | surface epithelial cells | 0 |
| **Vulva/anal skin** | surface epithelial cells | 0 |

Table 2 shows the GPX2 protein expression pattern in 216 spots representing 20 different cancer types. GPX2 expression was observed in a subset of adenocarcinomas, e.g. stomach, pancreas and colorectal carcinomas, and in urothelial cancers and liver cancers. Interestingly, the GPX2 protein expression ranges from 0 to 3 within each of these cancer types. Most other tumor types, e.g. squamous cell carcinomas, malignant gliomas and lymphomas, showed weak or no staining. In colorectal carcinomas, 10 out of 12 samples showed GPX2 protein expression. Three of these samples showed strong cytoplasmic positivity and the remaining 7 samples showed moderate positivity. Five out of 12 urothelial cancers showed cytoplasmic GPX2 expression. One sample showed strong positivity and 3 cases showed moderate positivity. The remaining 8 cases of urothelial cancer showed weak or no staining. The GPX2 expression also varied (from 3 to 0) within the groups of subjects suffering from liver cancer, pancreatic cancer and stomach cancer, respectively.

| **Table 2:** Expression pattern of GPX2 in 20 cancer types | | | | | | | | | | | | |
|---|---|---|---|---|---|---|---|---|---|---|---|---|
| **Cancer type** | **Subject number** | | | | | | | | | | | |
| | **1** | **2** | **3** | **4** | **5** | **6** | **7** | **8** | **9** | **10** | **11** | **12** |
| Breast cancer | 2 | 1 | 0 | 0 | 0 | 0 | 0 | 0 | 0 | 0 | 0 | N.R. |
| Cervical cancer | 1 | 0 | 0 | 0 | 0 | 0 | 0 | 0 | 0 | 0 | 0 | N.R. |
| Colorectal cancer | 3 | 3 | 3 | 2 | 2 | 2 | 2 | 2 | 2 | 2 | 0 | 0 |
| Endometrial cancer | 1 | 0 | 0 | 0 | 0 | 0 | 0 | 0 | 0 | 0 | 0 | N.R. |
| Head & neck cancer | 1 | 0 | 0 | 0 | | | | | | | | |
| Liver cancer | 3 | 2 | 2 | 1 | 1 | 1 | 1 | 1 | 0 | 0 | N.R. | N.R. |
| Lung cancer | 2 | 1 | 1 | 1 | 0 | 0 | 0 | 0 | 0 | 0 | 0 | 0 |
| Malignant carcinoid | 0 | 0 | 0 | 0 | | | | | | | | |
| Malignant glioma | 0 | 0 | 0 | 0 | 0 | 0 | 0 | 0 | 0 | 0 | N.R. | N.R. |
| Malignant lymphoma | 1 | 1 | 0 | 0 | 0 | 0 | 0 | 0 | 0 | 0 | 0 | 0 |
| Malignant melanoma | 2 | 1 | 0 | 0 | 0 | 0 | 0 | 0 | 0 | 0 | 0 | 0 |
| Ovarian cancer | 1 | 0 | 0 | 0 | 0 | 0 | 0 | 0 | 0 | 0 | 0 | 0 |
| Pancreatic cancer | 3 | 3 | 2 | 2 | 2 | 1 | 1 | 1 | 1 | 0 | 0 | 0 |
| Prostate cancer | 0 | 0 | 0 | 0 | 0 | 0 | 0 | 0 | 0 | 0 | 0 | 0 |
| Renal cancer | 2 | 1 | 0 | 0 | 0 | 0 | 0 | 0 | 0 | 0 | 0 | N.R. |
| Skin cancer | 0 | 0 | 0 | 0 | 0 | 0 | 0 | 0 | 0 | 0 | 0 | 0 |
| Stomach cancer | 3 | 2 | 1 | 1 | 1 | 1 | 1 | 0 | 0 | 0 | 0 | N.R. |
| Testis cancer | 2 | 1 | 0 | 0 | 0 | 0 | 0 | 0 | 0 | 0 | 0 | 0 |
| Thyroid cancer | 2 | 0 | 0 | 0 | | | | | | | | |
| Urothelial cancer | 3 | 2 | 2 | 2 | 1 | 0 | 0 | 0 | 0 | 0 | 0 | 0 |

### 4) Colorectal cancer TMA

### a) Material and methods

Archival formalin-fixed paraffin-embedded tissue from 122 patients (63 women and 59 men) diagnosed with colorectal carcinoma between 1999 and 2002 was collected from the Department of Pathology, Malmö University Hospital, Sweden. The median age of patients was 75 (32-88) years. 39 tumors were Dukes' stage A, 42 Dukes' stage B and 41 Dukes' stage C. Information regarding the date of death was obtained from the regional cause-of-death registries for all patients. Ethical permission was obtained from the Local Ethics Committee.

All 122 cases of colorectal carcinoma were histopathologically reevaluated on slides stained with hematoxylin and eosin. TMA:s were then constructed by sampling 2 x 1.0 mm cores per case from areas representative of invasive cancer. The TMA:s were prepared and automated immunohistochemistry was performed as described in section 3 above, using the GPX2 antibody prepared as described in section 2 above.

Tissue annotation was essentially done as described in section 3 above, with the exception that staining intensity and fraction of stained cells were not combined to yield a "staining score". Cytoplasmic intensity and cytoplasmic fraction were instead analyzed separately for correlation with survival. "Cytoplasmic intensity" corresponds to the over-all staining intensity of cytoplasms of glandular cells in a sample. "Cytoplasmic fraction" corresponds to the percentage of glandular cells in a sample that exhibits a positive staining intensity in the cytoplasm, wherein moderate or strong immunoreactivity is considered positive, and no or faint immunoreactivity is considered negative.

Based on the survival trends for all different strata, a dichotomized variable was constructed for further statistical analyses. From this analysis, the cytoplasmic intensity was divided into no immunoreactivity (0) or faint, moderate or strong immunoreactivity (>0). Further, the cytoplasmic fraction was divided into <=25% positive cells (<=1), i.e. 25 % or less of cells in a sample that exhibited a positive staining, or >25% positive cells (>1), i.e. more than 25 % of cells in a sample that exhibits a positive staining.

The above classification of samples was used for overall survival analysis according to the Kaplan-Meier estimator, and the log-rank test was used to compare survival in different strata. All statistical tests were two-sided, and p-values of < 0.05 % were considered significant. All calculations were made with the statistical package SPSS 12.0 (SPSS Inc. Illinois, USA).

### b) Results

Tissue microarray based analysis of 120 (122 subjects were analyzed but 2 of these were lacking representative tissue) colorectal carcinomas showed that 110 subjects (90 %) were positive for GPX2 as seen in figure 1. The figure further reveals that for 85% of the subjects, fractions of stained cells are >25%. The intensity was in most cases weak to moderate (87.5%). Survival analysis based on the cytoplasmic fraction or cytoplasmic intensity for the entire cohort revealed a significantly (p<0.001) lower five year overall survival (OS) for patients having tumors with no or low GPX2 expression (figure 2A and 2B). With regard to cytoplasmic fraction, the overall five-year survival was about 25 % for patients with 25 % or less cells positive for GPX2 (CF<=1) and about 67 % for patients with more than 25 % cells positive for GPX2 (CF>1) (figure 2A). A similar trend is observed for cytoplasmic intensity: the overall five-year survival was about 66 % for patients with faint, moderate or strong immunoreactivity (CI>0) and about 23 % for patients with no immunoreactivity (CI=0) (figure 2B). Dividing the cohort into Dukes' stages A, B and C revealed that a lost expression of GPX2 was associated with decreased overall survival for each of these stages (figures 3A-3C). A particularly significant trend was observed for Dukes' stages B and C, respectively. However, the most significant results were observed when combining data from Dukes' stages A and B (p=0.01) (figure 3D).

As can be seen from figure 2A and 2B, in a patient group diagnosed with colorectal cancer, the GPX2 protein expression clearly correlates with survival, such as five-year survival. It is reasonable to assume that a correspondent correlation applies to other types of cancers, especially to cancer types exhibiting varying GPX2 protein expression among patients diagnosed with that cancer type. Examples of such cancer types, e.g. liver cancer, pancreatic cancer, stomach cancer and urothelial cancer, are seen in table 2 above.

### 5) Urothelial cancer TMA

### a) Material and methods

Archival formalin-fixed, paraffin-embedded tissue from 190 patients (distribution women:men 1:3) diagnosed with urinary bladder carcinoma between 1980 and 2000 was collected from the Department of Pathology, Uppsala University Hospital, Sweden. No information regarding age and date of death is currently available. Ethical permission was obtained from the Local Ethics Committee. From these patients, 190 cores were histologically reevaluated on slides stained with hematoxylin and eosin.

Three TMAs were constructed by sampling from areas representative for respective category according to below. TMA no 1 was constructed by sampling of 2 x 0.6 mm cores per case, in total 2 x 117 cores, from areas representing superficial bladder carcinoma (Ta), non invasive (T1 non inv) and submucosal invasive cancer (T1 inv), originating from 97 patients. Out of the 45 Ta patients, 20 patients were also diagnosed with concomitant submucosal invasive cancer (T1 inv), from which an additional 2 x 0.6 mm cores per case were included. From a further 7 patients, samples of the T1 inv stage were taken and included on the TMA. The superficial tumors were in general of low to moderate malignancy grade, whereas the submucosal invasive tumors were of moderate to high malignancy grade. TMA no 2 was constructed by sampling of 2 x 1.0 mm cores per case, in total 2 x 56 cores, from areas representing muscle invasive bladder carcinoma, i.e. stages T2, T3 and T4, originating from 56 patients. In the majority of these cases, the malignancy grade was high. TMA no 3 was constructed by sampling of 4 x 1.0 mm cores per case from patients representing metastasizing bladder carcinoma. Matching samples were collected from primary tumor and site of metastasis from 20 patients. In addition, duplicate samples of morphologically normal urothelium from 17 patients were included. In total 2 x 57 cores where included on the array. No information on malignancy grade for the metastasizing bladder carcinomas is currently available. When the TMA:s were prepared, automated immunohistochemistry was performed as described in section 3 above, using the GPX2 antibody prepared as described in section 2 above.

Tissue annotation was essentially done as described in sections 3 and 4 above, but only cytoplasmic intensity was analyzed. Thereafter the subjects where separated into groups based on TNM stages and the average of cytoplasmic intensity for each stage were calculated. Moreover, the relative abundance of different cytoplasmic intensity categories for each stage was calculated.

### b) Results

Tissue microarray based analysis of urinary bladder carcinomas, reveals that 169 of the 190 subjects could be analyzed (not including the data from 20 patients with metastasizing bladder carcinoma), i.e. were showing representative tissue. Of these 169 subjects, 130 (77 %) were positive for GPX2 as seen in figure 4, and the dominating staining intensity was moderate (36 %). Stage analysis based on the cytoplasmic intensity revealed that the signal is steadily decreasing from normal tissue to T4 (figure 5A). In figure 5B, the relative abundance of different cytoplasmic intensities for each stage is demonstrated and this graph reveals that in all normal subjects the signal is strong but already at the Ta stage some subjects have lost the signal. Stage analysis based on the cytoplasmic intensity for the primary tumors and corresponding metastases revealed that the signal is lower in metastasis samples than in primary tumor (figure 6A). In figure 6B, the relative abundance of different cytoplasmic intensities are demonstrated and this reveals that in primary tumors a majority of samples has a moderate signal whereas in corresponding metastases the signal is generally weaker.

Consequently, GPX2 expression correlates with the TNM stages: the GPX2 protein expression is lower for cancers in more advanced TNM stages. Further, the survival, such as the five-year survival, decreases through the TNM stages. Accordingly, GPX2 expression correlates with survival of urothelial cancer, and GPX2 protein may be used as a marker in urothelial cancer prognosis.

### 6) Cancer prognostics

A non-limiting example of an establishment of a cancer prognosis is outlined below.

A cancer patient can present symptoms or signs from a tumor growth, focal symptoms including pain and distress from the region where the tumor grows or more general symptoms such as weight loss and fatigue. Signs from tumor growth can also become evident through bleeding (e.g., for colorectal cancer: blood in feces; and for urothelial cancer: blood in urine) and/or dysfunction (e.g., for colorectal cancer: diarrhea/constipation; and for urothelial cancer: frequent urination/stinging).

In addition, screening may be performed to detect asymptomatic tumors. Depending on symptoms and signs, a preliminary diagnose is made based on thorough questioning of patient history and clinical examination. To further establish the preliminary diagnose, the patient is investigated by blood tests (laboratory screens to assess the patients general condition) and endoscopic examination (recto- and colonoscopy for bowel disease and cystoscopy for urinary disease). Optionally, radiological methods to map extent of tumor growth and search for metastasis are also performed.

Following the finding of a suspected tumor, a biopsy is performed. A biopsy is the removal of a selected physical piece of tissue from the suspected tumor. Biopsies are preformed as part of the endoscopic procedure or can be performed using open surgery or mid- or coarse core biopsy tools (often guided by ultrasound). When applicable a cytological sample can also be obtained by fine needle aspiration of cells from the suspected mass or by the collection of fluids, e.g. urine, to detect presence of tumor cells in the cytological sample. The biopsy material is fixated in buffered formalin and histo-processed in order to obtain a thin section (4 µm) of the biopsy material. The tissue section may be histochemically stained with to facilitate microscopic examination. Subsequently, an experienced person, most typically a pathologist, distinguishes normal cells from cancer cells in the microscope to obtain a cancer diagnose. Following a cancer diagnose, the tissue section may be immunohistologically stained to analyze GPX2 protein expression, using the GPX2 specific antibodies and labeled secondary antibodies of Examples of the present disclosure, for obtaining prognosis information. In addition, other immunohistochemical staining or other molecular pathological methods may be used to further characterize the tumor. The clinical investigation may be expanded using various radiological methods to obtain staging information.

The surgical procedure to remove the tumor is then planned and eventual pre-operative treatment given depending on diagnose and additional findings. Subsequent to surgical removal of the tumor and surrounding tissues, the specimen is sent to a pathology laboratory, typically in buffered formalin, for histo-pathological examination.

Gross examination is performed by the pathologist in a procedure aiming to evaluate the extent of tumor growth, including collection and sampling of lymph nodes present in the specimen as well as taking samples from the resection margins to enable an analysis of radical excision of the tumor. Samples from representative tumor, resection margins and lymph nodes are embedded in paraffin blocks and sections are cut for microscopy. A pathologist will then analyze the histochemically stained sections, and thereafter make a diagnosis of the tumor and make a prognosis for the patient. The diagnose describes the tumor type and extent of tumor growth, including information on tumor stage, all of which yields important basic prognostic information. An immunohistochemical method performed according to claim 1 of the present disclosure is used to obtain further prognostic information: an obtained section is prepared and stained using GPX2 protein specific antibodies and secondary antibodies according to Examples, sections 1-3; a sample value is determined based on visual inspection of the stained section, and the determination is aided by visual inspection of a reference sample having a high amount of GPX2 protein expression, such as sample of normal urothelial tissue, and another reference sample having essentially no GPX2 protein expression, both reference samples being stained in the same way as the obtained section; as a mental act, the sample value is compared to a reference value which is 0, corresponding to essentially no GPX2 expression; and, if the sample value also is 0, corresponding to essentially no GPX2 protein expression, a conclusion is drawn that the prognosis is worse than, or equal to, a reference prognosis being associated with the reference value 0. For example, in the case where the above diagnosis has revealed a colorectal cancer, the reference prognosis may be a likelihood of five-year survival of 65 % (see figure 2B, CI>0 corresponds to a likelihood of five year survival of 66 %, and consequently, CI=0 is associated with a prognosis which is lower than 66 %, such as 65 % or lower). In further immunohistochemical staining, tumor or cell type-specific markers may be used to detect minute tumor spread in lymph nodes or proliferation markers to distinguish malignant cells from normal cells. The final results of gross and microscopic analysis, including immunohistochemistry, are summarized and the report is presented to the doctor responsible for the cancer patient. This report forms an important fundament for further management and treatment of the patient.

## Claims

**1.** Method for determining whether a prognosis for a mammalian subject having or suspected of having a cancer is worse than or equal to a reference prognosis, comprising the steps of:
a) providing a sample earlier obtained from the subject;
b) evaluating the amount of GPX2 protein present in at least part of said sample, and determining a sample value corresponding to said amount;
c) comparing the sample value obtained in step b) with a reference value associated with said reference prognosis; and, if said sample value is equal to or lower than said reference value,
d) concluding that the prognosis for said subject is equal to or worse than said reference prognosis.

**2.** Method of treatment of a subject being in need thereof, wherein the subject is having a cancer, comprising:
a) providing a sample from the subject;
b) evaluating the amount of GPX2 protein present in at least part of said sample, and determining a sample value corresponding to said amount;
c) comparing the sample value obtained in step b) with a reference value associated with a reference prognosis; and, if said sample value is equal to or lower than said reference value,
d) treating said subject with a treatment regimen adapted to a prognosis which is worse than or equal to the reference prognosis.

**3.** Method according to claim 2, in which said treatment regimen is selected from chemotherapy, neo-adjuvant therapy and combinations thereof.

**4.** Method according to claim 3, in which said treatment regimen is neo-adjuvant therapy.

**5.** Method according to claim 4, in which said neo-adjuvant therapy is selected from i) radiation therapy only and ii) radiation therapy in combination with chemotherapy.

**6.** Method according to any preceding claim, in which the sample is a body fluid sample.

**7.** Method according to claim 6, in which the body fluid is selected from the group consisting of blood, plasma, serum, cerebral fluid, urine, semen and exudate.

**8.** Method according to any one of claims 1-5, wherein said sample is a tissue sample.

**9.** Method according to claim 8, wherein the tissue sample is derived from the gastrointestinal tract including accessory organs of said subject.

**10.** Method according to any one of claims 8-9, wherein the tissue sample is a colorectal tissue sample.

**11.** Method according to any one of claims 1-5, in which the sample is a cytology sample.

**12.** Method according to any one of claims 1-5, in which the sample is a stool sample.

**13.** Method according to any preceding claim, wherein said sample comprise epithelial cells from said subject.

**14.** Method according to claim 13, wherein said sample comprise glandular cells from said subject.

**15.** Method according to claim 13, wherein said sample comprise transitional cells from said subject.

**16.** Method according to any one of claims 8-10 and 13, wherein the evaluation of step b) is limited to evaluating the amount of GPX2 expression in the cytoplasm of epithelial cells of said sample.

**17.** Method according to any preceding claim, wherein said cancer is a malignant epithelial tumor.

**18.** Method according to any preceding claim, wherein said cancer is a malignant tumor in the gastrointestinal tract including accessory organs.

**19.** Method according to any preceding claim, wherein said cancer is a colorectal cancer.

**20.** Method according to any preceding claim, wherein said cancer is a Dukes' stage A colorectal cancer.

**21.** Method according to any one of claims 1-19, wherein said cancer is a Dukes' stage B colorectal cancer.

**22.** Method according to any one of claims 1-19, wherein said cancer is a Dukes' stage C colorectal cancer.

**23.** Method according to any one of claims 1-9 and 11-18, wherein said cancer is a pancreatic cancer.

**24.** Method according to any one of claims 1-9 and 11-18, wherein said cancer is a liver cancer.

**25.** Method according to any one of claims 1-9 and 11-18, wherein said cancer is a malignant tumor in the gastrointestinal tract.

**26.** Method according to claim 1-9, 11-18 and 25, wherein said cancer is a stomach cancer.

**27.** Method according to any one of claims 1-8 and 11-17, wherein said cancer is urothelial cancer.

**28.** Method according to any one of claims 1-8 and 11-17, wherein said cancer is selected from the group consisting of colorectal cancer, stomach cancer, pancreatic cancer, liver cancer and urothelial cancer.

**29.** Method according to claim 1-9, 11-18 and 28, wherein said cancer is selected from the group consisting of colorectal cancer, stomach cancer, pancreatic cancer, liver cancer.

**30.** Method according to any preceding claim, wherein said reference value is a predetermined value corresponding to the amount of GPX2 protein expression in a reference sample.

**31.** Method according to any preceding claim, wherein the sample value of step b) is determined as being either 1, corresponding to detectable GPX2 protein in the sample, or 0, corresponding to essentially no detectable GPX2 protein in the sample.

**32.** Method according to any preceding claim, wherein the reference value of step c) corresponds to a reference sample having essentially no GPX2 protein expression.

**33.** Method according to any preceding claim, wherein the reference value of step c) is 0.

**34.** Method according to any one of claims 1-30, wherein said reference value is a cytoplasmic fraction of 50 % GPX2 positive cells, or lower.

**35.** Method according to any one of claims 1-30 and 34, wherein said reference value is a cytoplasmic fraction of 25 % GPX2 positive cells, or lower.

**36.** Method according to any one of claims 1-30, wherein said reference value is a moderate cytoplasmic intensity of GPX2 protein expression, or lower.

**37.** Method according to any one of claims 1-30 and 36, wherein said reference value is no cytoplasmic intensity of GPX2 protein expression.

**38.** Method according to any one of claims 1-30, wherein said reference prognosis is a likelihood of five-year survival of 65 % or lower.

**39.** Method according to any one of claims 1-30, wherein said reference prognosis is the average five-year survival for the cancer at the time of performing the method.

**40.** Method according to any preceding claim, wherein the amino acid sequence of the GPX2 protein comprises a sequence selected from:
i) SEQ ID NO:1; and
ii) a sequence which is at least 85 % identical to SEQ ID NO:1.

**41.** Method according to any preceding claim, wherein the amino acid sequence of the GPX2 protein comprises a sequence selected from:
i) SEQ ID NO:2; and
ii) a sequence which is at least 85 % identical to SEQ ID NO:2.

**42.** Method according to any preceding claim, wherein the evaluation of step b) comprises:
bI) applying to the sample a quantifiable affinity ligand capable of selective interaction with the GPX2 protein to be quantified, said application being performed under conditions that enable binding of the affinity ligand to any GPX2 protein present in the sample;
bII) removing non-bound affinity ligand; and
bIII) quantifying any affinity ligand remaining in association with the sample.

**43.** Method according to claim 42, wherein the quantifiable affinity ligand is selected from the group consisting of antibodies, fragments thereof and derivatives thereof.

**44.** Method according to claim 42, wherein the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.

**45.** Method according to claim 42, wherein the quantifiable affinity ligand is an oligonucleotide molecule.

**46.** Method according to claim 42-45, wherein the quantifiable affinity ligand comprises a label selected from the group consisting of fluorescent dyes and metals, chromophoric dyes, chemiluminescent compounds and bioluminescent proteins, enzymes, radioisotopes and particles.

**47.** Method according to any one of claims 42-46, in which said quantifiable affinity ligand is detected using a secondary affinity ligand capable of recognizing the quantifiable affinity ligand.

**48.** Method according to claim 47, in which said secondary affinity ligand capable of recognizing the quantifiable affinity ligand comprises a label selected from the group consisting of fluorescent dyes and metals, chromophoric dyes, chemiluminescent compounds and bioluminescent proteins, enzymes, radioisotopes and particles.

**49.** Method according to any previous claim, wherein said subject is a human.

**50.** Method according to claim 1, which, if said sample value is higher than said reference value, further comprises a step of:
e) concluding that the prognosis for said subject is better than said reference prognosis.

**51.** Kit for carrying out the method according to any preceding claim, which comprises
a) a quantifiable affinity ligand capable of selective interaction with a GPX2 protein; and
b) reagents necessary for quantifying the amount of the affinity ligand.

**52.** Kit according to claim 51, in which the quantifiable affinity ligand is selected from the group consisting of antibodies, fragments thereof and derivatives thereof.

**53.** Kit according to claim 51, in which the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.

**54.** Kit according to claim 51, wherein the quantifiable affinity ligand is an oligonucleotide molecule.

**55.** Kit according to any one of claims 51-54, in which the quantifiable affinity ligand comprises a label selected from the group consisting of fluorescent dyes and metals, chromophoric dyes, chemiluminescent compounds and bioluminescent proteins, enzymes, radioisotopes and particles.

**56.** Kit according to any one of claims 51-54, in which said reagents necessary for quantifying the amount of the affinity ligand comprise a secondary affinity ligand capable of recognizing the quantifiable affinity ligand.

**57.** Kit according to claim 56, in which said secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes and particles.

**58.** Kit according to any one of claims 51-57, further comprising a reference sample for provision of the reference value.

**59.** Kit according to claim 58, wherein said reference sample is a tissue sample with essentially no GPX2 protein present.

**60.** Kit according to claim 58, wherein the reference sample contains an amount of GPX2 protein corresponding to a cytoplasmic fraction of 50 % GPX2 positive cells or lower.

**61.** Kit according to claim 58 or 60, wherein the reference sample contains an amount of GPX2 protein corresponding to a cytoplasmic fraction of 25 % GPX2 positive cells or lower.

**62.** Kit according to claim 58, wherein the reference sample contains an amount of GPX2 protein corresponding to a moderate cytoplasmic intensity of GPX2 expression or lower.

**63.** Kit according to claim 58 or 62, wherein the reference sample contains an amount of GPX2 protein corresponding to no cytoplasmic intensity of GPX2 expression.

**64.** Kit according to claim 58, wherein the reference sample contains an amount of GPX2 protein corresponding to a value being associated with a prognosis which is better than said reference prognosis.

**65.** Kit according to claim 58 and 64, further comprising a reference sample containing an amount of GPX2 protein corresponding to a strong cytoplasmic intensity.

**66.** Kit according to claim 58 and 64, further comprising a reference sample containing an amount of GPX2 protein corresponding to a cytoplasmic fraction of at least 75 % GPX2 positive cells.

**67.** Kit according to any one of claims 51-66, wherein the reference sample is a tissue sample.

**68.** Use of a GPX2 protein as a prognostic marker.

**69.** Use of a GPX2 protein as a prognostic marker for a cancer.

**70.** Use according to claim 69, wherein said cancer is a malignant epithelial tumor.

**71.** Use according to any one of claims 69-70, wherein said cancer is colorectal cancer.

**72.** Use of a GPX2 protein, or an antigenically active fragment thereof, in the manufacture of a prognostic agent for establishing a prognosis for patient having or suspected of having a cancer.

**73.** Use according any one of claims 68-72, wherein the amino acid sequence of the GPX2 protein comprises a sequence selected from:
i) SEQ ID NO:1; and
ii) a sequence which is at least 85 % identical to SEQ ID NO:1.

**74.** Use according any one of claims 68-73, wherein the amino acid sequence of the GPX2 protein comprises a sequence selected from:
i) SEQ ID NO:2; and
ii) a sequence which is at least 85 % identical to SEQ ID NO:2.

**75.** GPX2 protein for establishing a prognosis for a mammalian subject having or suspected of having a cancer.

**76.** GPX2 protein according to claim 75, wherein the amino acid sequence of the GPX2 protein comprises a sequence selected from:
iii) SEQ ID NO:1; and
iv) a sequence which is at least 85 % identical to SEQ ID NO:1.

**77.** GPX2 protein accprding to claim 75, wherein the amino acid sequence of the GPX2 protein comprises a sequence selected from:
i) SEQ ID NO:2; and
ii) a sequence which is at least 85 % identical to SEQ ID NO:2.

**78.** Affinity ligand capable of selective interaction with a GPX2 protein, which is an antibody or a fragment or a derivative thereof.

**79.** Affinity ligand according to claim 78, which is obtainable by a process comprising a step of immunizing an animal with a protein whose amino acid sequence comprises the sequence SEQ ID NO:1.

**79.** Affinity ligand according to any one of claims 78 and 79 for establishing a prognosis for a mammalian subject having a cancer.

**80.** Use of an affinity ligand according to any one of claims 78 and 79 as a prognostic agent.

**81.** Use of an affinity ligand according to any one of claims 78 and 79 establishing a prognosis for a mammalian subject having a cancer.

**82.** Use of a an affinity ligand according to any one of claims 78 and 79 in the manufacture of a prognostic agent for establishing a prognosis for a mammalian subject having a cancer.


SEQUENCE LISTING

<110>  Atlas Antibodies

<120>  PROGNOSTIC METHOD

<130>  21032829

<160>  3

<170>  PatentIn version 3.3

<210>  1
<211>  89
<212>  PRT
<213>  Homo sapiens

<400>  1

Val Leu Gly Phe Pro Cys Asn Gln Phe Gly His Gln Glu Asn Cys Gln
1               5                   10                  15

Asn Glu Glu Ile Leu Asn Ser Leu Lys Tyr Val Arg Pro Gly Gly Gly
            20                  25                  30

Tyr Gln Pro Thr Phe Thr Leu Val Gln Lys Cys Glu Val Asn Gly Gln
        35                  40                  45

Asn Glu His Pro Val Phe Ala Tyr Leu Lys Asp Lys Leu Pro Tyr Pro
    50                  55                  60

Tyr Asp Asp Pro Phe Ser Leu Met Thr Asp Pro Lys Leu Ile Ile Trp
65                  70                  75                  80

Ser Pro Val Arg Arg Ser Asp Val Ala
                85

<210>  2
<211>  190
<212>  PRT
<213>  Homo sapiens

<400>  2

Met Ala Phe Ile Ala Lys Ser Phe Tyr Asp Leu Ser Ala Ile Ser Leu
1               5                   10                  15

Asp Gly Glu Lys Val Asp Phe Asn Thr Phe Arg Gly Arg Ala Val Leu
            20                  25                  30

Ile Glu Asn Val Ala Ser Leu Sec Gly Thr Thr Thr Arg Asp Phe Thr
        35                  40                  45

Gln Leu Asn Glu Leu Gln Cys Arg Phe Pro Arg Arg Leu Val Val Leu
    50                  55                  60

Gly Phe Pro Cys Asn Gln Phe Gly His Gln Glu Asn Cys Gln Asn Glu
65                  70                  75                  80

Glu Ile Leu Asn Ser Leu Lys Tyr Val Arg Pro Gly Gly Gly Tyr Gln
                85                  90                  95

Pro Thr Phe Thr Leu Val Gln Lys Cys Glu Val Asn Gly Gln Asn Glu
            100                 105                 110

His Pro Val Phe Ala Tyr Leu Lys Asp Lys Leu Pro Tyr Pro Tyr Asp
        115                 120                 125

Asp Pro Phe Ser Leu Met Thr Asp Pro Lys Leu Ile Ile Trp Ser Pro
    130                 135                 140

Val Arg Arg Ser Asp Val Ala Trp Asn Phe Glu Lys Phe Leu Ile Gly
145                 150                 155                 160

Pro Glu Gly Glu Pro Phe Arg Arg Tyr Ser Arg Thr Phe Pro Thr Ile
                165                 170                 175

Asn Ile Glu Pro Asp Ile Lys Arg Leu Leu Lys Val Ala Ile
            180                 185                 190

<210>  3
<211>  573
<212>  DNA
<213>  Homo sapiens

<400>  3
atggctttca ttgccaagtc cttctatgac ctcagtgcca tcagcctgga tggggagaag      60

gtagatttca atacgttccg gggcagggcc gtgctgattg agaatgtggc ttcgctctga     120

ggcacaacca cccgggactt cacccagctc aacgagctgc aatgccgctt tcccaggcgc     180

ctggtggtcc ttggcttccc ttgcaaccaa tttggacatc aggagaactg tcagaatgag     240

gagatcctga acagtctcaa gtatgtccgt cctgggggtg gataccagcc caccttcacc     300

cttgtccaaa aatgtgaggt gaatgggcag aacgagcatc ctgtcttcgc ctacctgaag     360

gacaagctcc cctaccctta tgatgaccca ttttccctca tgaccgatcc caagctcatc     420

atttggagcc ctgtgcgccg ctcagatgtg gcctggaact ttgagaagtt cctcataggg     480

ccggagggag agcccttccg acgctacagc cgcaccttcc caaccatcaa cattgagcct     540

gacatcaagc gcctccttaa agttgccata tag                                  573